**Europäisches Patentamt**

**European Patent Office**

**Office européen des brevets**

(19)

(11)  **EP 1 141 677 B1**

(12)  **EUROPÄISCHE PATENTSCHRIFT**

(45) Veröffentlichungstag und Bekanntmachung des Hinweises auf die Patenterteilung:
**31.07.2002 Patentblatt 2002/31**

(51) Int Cl.[7]: **G01N 21/35**, G01N 33/28

(21) Anmeldenummer: **00902551.1**

(22) Anmeldetag: **04.01.2000**

(86) Internationale Anmeldenummer:
**PCT/DE00/00067**

(87) Internationale Veröffentlichungsnummer:
**WO 00/40948 (13.07.2000 Gazette 2000/28)**

(54) **GASQUALITÄTSBESTIMMUNG**

METHOD OF DETERMINING THE GAS QUALITY

PROCEDE POUR DETERMINER LA QUALITE D'UN GAZ

(84) Benannte Vertragsstaaten:
**AT BE CH CY DE DK ES FI FR GB GR IE IT LI LU MC NL PT SE**

(30) Priorität: **05.01.1999 DE 19900129**

(43) Veröffentlichungstag der Anmeldung:
**10.10.2001 Patentblatt 2001/41**

(73) Patentinhaber: **Flowcomp Systemtechnik GmbH 44357 Dortmund (DE)**

(72) Erfinder:
- **STIRNBERG, Dieter**
**D-44265 Dortmund (DE)**
- **KASTNER, Joachim**
**D-44139 Dortmund (DE)**

(74) Vertreter: **Schneider, Uwe, Dipl.-Ing.**
**Patentanwalt**
**Holbeinstrasse 27**
**59423 Unna (DE)**

(56) Entgegenhaltungen:
**EP-A- 0 708 323** **US-A- 3 950 101**
**US-A- 4 594 510** **US-A- 4 958 076**
**US-A- 5 822 058**

- **BUONANNO G ET AL: "The influence of reference condition correction on natural gas flow measurement" MEASUREMENT,GB,INSTITUTE OF MEASUREMENT AND CONTROL. LONDON, Bd. 23, Nr. 2, 1. März 1998 (1998-03-01), Seiten 77-91, XP004123932 ISSN: 0263-2241**

**Beschreibung**

Die Erfindung betrifft Verfahren zur photometrischen Bestimmung der Gasqualität, insbesondere von Brenngasen, gemäß Oberbegriff der Ansprüche 1 und 8 sowie eine Vorrichtung zur photometrischen Bestimmung der Gasqualität, insbesondere von Brenngasen, gemäß Oberbegriff des Anspruches 12.

Zur Erfassung der Gasbeschaffenheit zum Beispiel in Verteilungsnetzen für Erdgas oder dgl. werden schon seit langem Einrichtungen zur Erfassung der Beschaffenheit des jeweils durchgeleiteten Gases, sog. Gasbeschaffenheitsmeßgeräte benutzt. Erdgas unterliegt als Naturprodukt je nach Herkunft und durch Mischung entsprechenden Schwankungen bezüglich seiner Zusammensetzung, wobei die Zusammensetzung z.B. von Erdgas aus den verschiedenen Kohlenwasserstoffen aber wesentlich den Brennwert und daraus abgeleitete Größen bestimmt. Daher ist es für die Abrechnung der durch ein Gasversorgungsnetz durchgeleiteten Gasmenge und damit der entsprechenden Energiemenge von großer Wichtigkeit, die jeweilige Gasbeschaffenheit an der Einspeisestelle in das Erdgasnetz und der Abnahmestelle des Kunden genau zu erfassen und damit eine tatsächlich transportierte bzw, gelieferte Energiemenge zu bestimmen und abzurechnen. Somit kann dem Abnehmer des Gases auch bei sich unterscheidender Gasbeschaffenheit und einem entsprechend schwankendem Energiegehalt immer ein dem tatsächlich gelieferten Energiegehalt entsprechender Preis in Rechnung gestellt werden. Umgekehrt bietet die Erfassung der Gasbeschaffenheit dem Abnehmer die Gewähr, nachprüfbar eine von ihm verlangte Qualität und damit geforderten Energiegehalt geliefert zu erhalten.

Die Erfassung der Gasbeschaffenheit erhält zusätzliche Bedeutung, daß mit Fall des Durchleitungsmonopols der Erdgasversorger durch ein und dasselbe Versorgungsnetz Gase ganz unterschiedlicher Herkunft und damit auch unterschiedlicher Zusammensetzung durchgeleitet werden. Nur eine möglichst einfache und kostengünstige Erfassung der Gasbeschaffenheit durch entsprechend bereitgestellte kostengünstige Meßgeräte und Meßverfahren erlaubt dann eine nachvollziehbare und genaue Abrechnung.

Zur Bestimmung der Gasqualität müssen als maßgebliche Größen möglichst genau und die Veränderungen der Gasqualität erfassend der volumetrische Normbrennwert $H_{v,n}$, die Normdichte $\rho_n$ und die Kompressibilitätszahl K bestimmt werden.

In der Praxis wird für die energetische Abrechnung zunächst mittels Durchflußmeßgeräten das transportierte Gasvolumen $V_b$ unter Betriebsbedingungen (Druck $p_b$, Temperatur $T_b$) gemessen. Bei Kenntnis der Gasbeschaffenheit läßt sich die Kompressibilitätszahl K bestimmen, mit der das Gasvolumen $V_n$ unter Normbedingungen (Druck $p_n$, Temperatur $T_n$) berechnet wird.

$$V_n = \frac{p_b}{p_n}\frac{T_n}{T_b}\frac{1}{K}V_b$$

Durch Multiplikation dieses Normvolumens mit dem volumetrischen Brennwert $H_{v,n}$ unter Normbedingungen erhält man die transportierte Energiemenge Q:

$$Q = V_n H_{v,n}$$

Alternativ kann auch direkt das Betriebsvolumen $V_b$ mit dem Betriebsbrennwert $H_{v,b}$ multipliziert werden (Energiemeter).

Eine weitere wichtige Größe bei Erdgasanwendungen ist die thermische Leistungsabgabe von Gasbrennern; sie variiert ebenso mit der Gasbeschaffenheit und wird durch den sogenannten Wobbeindex $W_v$ charakterisiert: Gase mit gleichem Wobbeindex $W_v$ liefern die gleiche Wärmeleistung an einer Verbrennungsdüse. Zur Berechnung des Wobbeindexes $W_v$ ist die Normdichte $\rho_n$ des Gases erforderlich, aus der die relative Dichte bezüglich Luft bestimmt wird ($d_v = \rho_{Gas}/ \rho_{Luft}$)

$$W_v = \frac{Hv}{\sqrt{d_v}}$$

Daher kommt der Bestimmung des Normbrennwertes $H_{v,n}$ für die praktische Bestimmung der Gasqualität z. B. zu Abrechnungszwecken zentrale Bedeutung zu.

Bisher finden verschiedene Einrichtungen zur Erfassung der Gasbeschaffenheit Anwendung. Man unterscheidet hierbei sog. direkte und sog. indirekte Verfahren. Bei den direkten Verfahren werden die zu bestimmenden Größen einzeln gemessen und hierzu das Gas meist auf Normbedingungen gebracht, wodurch teilweise aufwendige Gasvorbehandlungen erforderlich werden.

Am einfachsten läßt sich die Gasbeschaffenheit mittels sog. Kalorimeter bestimmen, bei denen mittels einer offenen Flamme Probengas verbrannt und aus der entstehenden und an ein Kühlmedium abgegebenen Wärmemenge und der daraufhin feststellbaren Temperaturerhöhung des Kühlmediums der Brennwert des verbrannten Gases ermittelt wird. Derartige Geräte benötigen eine komplizierte Mechanik zur Einstellung eines bestimmten Mengenverhältnisses von Gas, Verbrennungsluft und z.B. Kühlluft als Kühlmedium und sind daher teuer und fehleranfällig, zumal durch die offene Verbrennung erhöhte Sicherheitsanforderungen an die Geräte zu stellen sind. Auch muß die Wartung und Kalibrierung von Fachpersonal durchgeführt werden, darüber hinaus müssen die Kalorimeter in konditionierten Räumen eingesetzt werden. Daher liegen; die Anschaffungs- und Betriebskosten derartiger Meßanordnungen sehr hoch.

Bei der Kalometrie durch katalytische Verbrennung (z. B. mit Pellistoren) wird das

Probengas mit Luft gemischt und an den 400 bis 500°C heißen Wendeln eines Katalysators verbrannt. Die Temperaturerhöhung des Katalysators ist dabei proportional zum Brennwert. Da dieses Verfahren auf einem empfindlichen Oberflächeneffekt basiert, ist es starken Driften unterworfen und erfordert häufige Kalibration mit Prüfgas. Die katalytischen Kalorimeter sind von allen hier beschriebenen Verfahren am günstigsten, allerdings eignen sie sich von ihrer Genauigkeit eher zur Kontrolle als zur Abrechnung.

Die direkte Messung der Betriebsdichte $\rho_b$ erfolgt zum einen mit Dichtewaagen, sehr aufwendigen Präzisionsgeräten, bei denen man den Auftrieb einer stickstoffgefüllten Kugel in Abhängigkeit der Dichte des umgebenden Mediums, hier des Probengases, mißt. Bei einem anderen Verfahren wird ein dünnwandiger Metallzylinder, der vom Probengas umströmt wird, in Schwingung versetzt. Die Dichte des umgebenden Gases bestimmt die Resonanzfrequenz des Zylinders, die als empfindliche Meßgröße erfaßt wird. Beide Verfahren sind für die Bestimmung der Normdichte sehr aufwendig, da sie die Einstellung des Normzustandes erfordern.

Die Kompressibilitätszahl K wird nun nicht direkt gemessen, sondern kann nach unterschiedlichen numerischen Standard-Berechnungsverfahren aus den direkt meßbaren Gasgrößen berechnet werden. Das eine Verfahren, das sog. GERG88-Verfahren (DVGW-Arbeitsblatt 486) benötigt dabei die in Tabelle 1 aufgeführten Eingangsgrößen. Der Stoffmengenanteil von $CO_2$ wird nach dem heutigen Stand der Technik durch ein nicht dispersives infrarotspektroskopisches Verfahren (NDIR) bestimmt, wobei das Gas in einen definierten Zustand nahe oder bei Normbedingung gebracht werden muß. Der Stoffmengenanteil von $H_2$ ist praktisch nur bei der Betrachtung von Kokereigasen von Bedeutung und kann in den heute in Europa verteilten typischen Erdgasen praktisch vernachlässigt werden. Die Kompressibilitäts-Zahl K läßt sich mit der GERG88-Gleichung bei genügender Genauigkeit der Eingangsgrößen auf $10^{-3}$ bestimmen.

Tabelle 1:

| Eingangsgrößen des GERG88-Verfahrens | |
|---|---|
| $P_b$ | Betriebsdruck |
| $T_b$ | Betriebstemperatur |
| $\rho_n$ | Dichte im Normzustand |
| $H_{v,n}$ | Volumetrischer Brennwert im Normzustand |
| $X_{CO_2}$ | Stoffmengenanteil $CO_2$ |
| $X_{H_2}$ | Stoffmengenanteil $H_2$ |

Das andere Verfahren zur Bestimmung des Realgasverhaltens erfolgt nach der AGA8-92DC-Gleichung (ISO 12213-2:1997 (E)). Dieses Verfahren benötigt als Eingangswerte die Stoffmengenanteile von 21 führenden Gaskomponenten (Tabelle 2) und erreicht ebenso eine Genauigkeit von $10^{-3}$.

Tabelle 2:

| Eingangsgrößen der AGA8-92DC-Gleichung | |
|---|---|
| Methan | $CO_2$ |
| Ethan | $N_2$ |
| Propan | $H_2S$ |
| Isobutan | He |
| n-Butan | $H_2O$ |

Tabelle 2: (fortgesetzt)

| Eingangsgrößen der AGA8-92DC-Gleichung | |
|---|---|
| Isopentan | $O_2$ |
| n-Pentan | Ar |
| n-Hexan | $H_2$ |
| n-Heptan | CO |
| n-Oktan | Druck |
| n-Nonan | Temperatur |
| n-Dekan | |

Der Stand der Technik umfaßt neben den direkten Meßtechniken auch die indirekte Gasbeschaffenheitsmessung mittels Gaschromatographie. Dabei wird ein definiertes Volumen des Probengases in einen definierten Zustand gebracht und von einem Trägergas, typischerweise Helium, durch ein System von gaschromatographischen Trennsäulen getragen. Aufgrund ihrer unterschiedlichen Retentionszeiten erreichen die einzelnen Gaskomponenten den nachgeschalteten Sensor, im allgemeinen ist dies ein Wärmeleitfähigkeitsdetektor, am Ende der Trennsäule zeitlich getrennt. Die Peakfläche des Sensorsignals kann nun als Stoffmenge interpretiert werden, wobei die Auswertung im Vergleich zu einem Referenzgas erfolgen muß, das in etwa ähnliche Zusammensetzung wie das Probengas haben muß. Der Nachteil der Gaschromatographie liegt in der aufwendigen Probenaufbereitung und Installation des Gesamtsystems, und in der aufwendigen Unterhaltung und Bedienung durch geschultes Personal. Aus den Stoffmengenanteilen der einzelnen Gaskomponenten, wie sie die Gaschromatographie liefert, lassen sich dann alle relevanten Gasgrößen berechnen. Für die Realisierung derartiger indirekter Messungen durch Chromatographie werden automatisch arbeitende Prozeßchromatographen mit Wärmeleitfähigkeitsdetektoren eingesetzt. Diese Geräte messen normalerweise elf Komponenten des Erdgases ($N_2$, $CO_2$, $CH_4$, $C_2H_6$, $C_3H_8$, $C_4H_{10}$, $C_5H_{12}$, $C_6$+, usw.). Als Trägergas wird Helium verwendet, wobei dessen leichte Flüchtigkeit in der Praxis oft zur vorzeitigen Entleerung der Trägergasflasche führt und daher kurze Wartungszyklen eines derartigen Gasbeschaffenheitsmeßgerätes bedingt. Als Kalibriergas wird ein Gas gewählt, das dem zu messenden Erdgas ähnlich ist. Derartige Chromatographensysteme führen ohne Unterbrechung Meßzyklen aus, damit Änderungen der Gasbeschaffenheit sofort erfaßt werden. Dieses führt zu einem hohen Verbrauch an Trägergas und Kalibriergas und hat zudem zur Folge, daß Wartungen des Gerätes in relativ kurzen Abständen durchgeführt werden müssen.

Es ist ebenfalls bekannt, mit üblichen Infrarot-Gasanalysatoren die Zusammensetzung eines Gases zu bestimmen. Derartige im mittleren Infrarot oder nahen Infrarot arbeitende Analysatoren bieten jedoch nicht die für eine Brennwertbestimmung erforderlichen hohen Genauigkeits- und insbesondere Stabilitätsanforderungen unter den hier erforderlichen Meßbedingungen. Auch ist neben der eigentlichen Messung an dem Probengas immer eine parallel laufende Referenzmessung erforderlich, um zumindest die wesentlichsten Fehlereinflüsse kompensieren zu können. Als Meßergebnisse liefern die bekannten Infrarot-Gasanalysatoren überlagerte Frequenzspektren, die eine Rückbeziehung auf einzelne Komponenten eines untersuchten Gases stark erschweren, wenn nicht sogar unmöglich machen.

In der Literatur wurde auch ein infrarotspektroskopisches Verfahren zur Gasanalyse beschrieben ("Optical BTU Sensor Development", Gas Research Institute GRI-93/0083), das mittels sog. Multivariater Analyse (MVA) der Nahinfrarotspektren von Gasen die volumetrische Stoffmengenkonzentration der kohlenstoffhaltigen Komponenten des Gases und damit den volumetrischen Brennwert unter Betriebsbedingungen ermittelt. Dieses Verfahren liefert jedoch nicht den Brennwert $H_{v,n}$ unter Normbedingungen und nicht die Normdichte pn und den Stoffmengenanteil von $CO_2$, so daß es nicht zur Bestimmung der Kompressibilitäts-Zahl K und damit zur vollständigen Gasqualitätsbestimmung geeignet ist. Bei der Bestimmung des Brennwertes mittels vorbekannter photometrischer Methoden werden zudem geringere Ansprüche an die notwendige apparative Ausstattung zur Durchführung der Verfahren gestellt, wobei ein Vorteil in der Geschwindigkeit der Erfassung der Absorptionsspektren des Erdgases im nahen oder mittleren infraroten Spektralbereich liegt. Das gesamte Absorptionsspektrum des Erdgases setzt sich dabei aus der Summe der Einzelspektren der im Gas vorliegenden Komponenten additiv zusammen und kann daher gemessen und mit Hilfe geeigneter Spektrenanalyseverfahren analysiert werden. Hierbei ist der ermittelte Anteil einer Absorption einer Komponente an dem gesamten Spektrum des Erdgases im wesentlichen dem Konzentrationsanteil dieser Komponente im Prüfgas gleich. Mit Kenntnis des Brennwertes der jeweiligen Komponente kann dann der Brennwert des gesamten Gasgemisches als Summenwert berechnet werden. Problematisch an diesem Verfahren der Spektrenanalyse ist jedoch die starke Überlappung von Absorptionsbanden unterschiedlicher Komponenten, die häufig zu ungenauen Ergebnissen führen und darüber hinaus einen hohen Rechenaufwand benötigen.

Ein weiteres infrarotspektroskopisches Verfahren nach der DE 198 38 301 A1 arbeitet als direkte Spektralauswertung (DSA) mit einer Spektralfunktion, mit der das Gasspektrum gefaltet wird. Das Verfahren erlaubt die Bestimmung des volumetrischen Brennwertes $H_{v,b}$ unter Betriebsbedingungen direkt aus dem Spektrum. Hierzu wird ausgenutzt, daß bei der Gasverbrennung die jeweils erzeugte Reaktionswärme auf der Verbrennung von C-H-Bindungen beruht und dabei eine erzeugte Wärmemenge von der vorliegenden Bindungsenergie abhängt. Dies wird dazu ausgenutzt, daß die Schwingungen der C-H-Bindungen, die eine zueinander gleiche, bestimmte Bindungsenergie aufweisen und bei einer Verbrennung die gleiche Wärmemenge erzeugen, bei einer zugeordneten Wellenlänge in Wechselwirkung mit einer elektromagnetischen Strahlung treten. Hierdurch ist durch wellenlängen-aufgelöstes Messen und einer wellenlängen-abhängigen Gewichtung der Wechselwirkungsgrade dieser Schwingungen die Berechnung des Brennwertes $H_{v,b}$ des Gases möglich, ohne daß eine Identifizierung einzelner Gaskomponenten erforderlich ist. Es wird dort darüber hinaus eine Vorrichtung zur Durchführung eines derartigen Verfahrens vorgeschlagen, die auf die spezifischen Erfordernisse des Meßverfahrens abgestimmt ist und eine gewichtete Aufsummierung der Wechselwirkungsgrade ermöglicht. Mit diesem Verfahren kann zwar der Brennwert $H_{v,b}$ eines Gasgemisches unter Betriebsbedingungen ermittelt werden, die anderen zur Gasqualitätsmessung benötigten Größen lassen sich jedoch meßtechnisch hiermit nicht erfassen.

In einer Veröffentlichung von G. Buonanno et al. "The influence of reference condition correction on natural gas flow measurement" in der britischen Zeitschrift "Measurement Bd. 23, Nr. 2 vom 1. März 1998 (herausgegeben vom Institue of Measurement and control, London) ist auf den Seiten 79 - 81 ein Verfahren zur Bestimmung der Gasvolumenkonvertierung angegeben, bei dem die Kompressibititätszahl Z gemäß dem Standard SGERG-88 iterativ bestimmt wird und danach die Parameter $\zeta$ und X berechnet werden. Diese Parameter sind mathematisch direkt mit den Eingangsgrößen Druck p, Temperatur T und den Stoffmengenantellen verbunden.

Weiterhin sind aus der US 495 80 76 und der US 582 20 58 photometrische Vorrichtungen mit Filtereinrichtungen bekannt.

Nach dem heutigen Stand der Technik gibt es kein, zumal kein einheitliches Verfahren, das unter Betriebsbedingungen die maßgeblichen Größen volumetrischer Norm-Brennwert $H_{v,n}$, Norm-Dichte $\rho_n$ und Kompressibilitäts-Zahl K ermittelt.

Es ist daher Aufgabe der vorliegenden Erfindung, Verfahren und Vorrichtungen für die Bestimmung der Gasqualität vorzuschlagen, bei dem die maßgeblichen Größen volumetrischer Norm-Brennwert $H_{v,n}$, Normdichte $\rho_n$ und Kompressibilitätszahl K anhand einer spektroskopischen Erfassung der Gasbeschaffenheit sowie entsprechenden Auswertungen bestimmt werden können.

Die Lösung der erfindungsgemäßen Aufgabe ergibt sich bezüglich der Verfahren aus den kennzeichnenden Merkmalen der Ansprüche 1 bzw. 8 in Zusammenwirken mit den Merkmalen des zugehörigen Oberbegriffes. Die Lösung der erfindungsgemäßen Aufgabe ergibt sich bezüglich der Vorrichtung aus den kennzeichnenden Merkmalen des Anspruches 12 in Zusammenwirken mit den Merkmalen des Oberbegriffes. Weitere vorteilhafte Ausgestaltungen der Erfindung ergeben sich aus den jeweiligen Unteransprüchen.

Die Erfindung betrifft ein erstes Verfahren gemäß Anspruch 1, bei dem die Bestimmung der Gasqualität eines Probengases, insbesondere eines Brenngases, ausgehend von einem unter Betriebsbedingungen mittels infrarotspektroskopischer Meßverfahren bestimmten Spektrums des Probengases vorgenommen wird. Hierbei wird in erfindungsgemäßer Weise die Gasqualität des Probengases dadurch bestimmt, daß in einem ersten Verfahrensschritt aus dem aufgenommenen Spektrum die Stoffmengenanteile $x_i$ der einzelnen Komponenten des Probengases im Betriebszustand bestimmt werden, daraufhin Vorgabewerte für Kompressibilitätszahl K und Realgasfaktor $Z_n$ zur Berechnung der gesuchten Kompressibilitätszahl K vorgegeben werden und dann aus gemessenen Betriebsgrößen des Probengases sowie den Stoffmengenanteilen $x_i$ und stoffspezifischen Größen, wie etwa den Brennwerten pro Molekülart der Komponenten und den entsprechenden Molekülmassen, und unter Einbeziehung der Vorgabewerte für Kompressibilitätszahl K und Realgasfaktor $Z_n$ die benötigten Eingangsgrößen für die Bestimmung der Kompressibilitätszahl K bestimmt werden. Diese Eingangsgrößen werden dazu benutzt, mittels Standard-Berechnungsverfahren die Kompressibititätszahl K zu berechnen. In einem weiteren Verfahrensschritt wird eine iterative Berechnung durch iterative Neuberechnung der Eingangsgrößen mit dem ermittelten Wert für die Kompressibilitätszahl K solange durchgeführt, bis der Wert der Kompressibilitätszahl K konvergiert. Daraus kann der volumetrische Normbrennwert $H_{v,n}$ und die Normdichte $\rho_n$ berechnet werden. Im Falle des Konvergierens liegen dann die benötigten Größen Kompressibilitätszahl K, Normbrennwert $H_{v,n}$ und Normdichte $\rho_n$ zur Bestimmung der Gasqualität eines Probengases unmittelbar vor und können entsprechend z.B. zur Berechnung des Energleinhaltes des transportierten Gases genutzt werden. Bei jeder Iteration wird daher der gerade ermittelte Wert der Kompressibilitätszahl K wieder in die Gleichungen zur Berechnung der Eingangsgrößen des Standard-Berechnungsverfahrens eingesetzt und ein neuer Iterationsschritt durchgeführt. Anhand dieses Verfahrens läßt sich aus der Bestimmung der Stoffmengenanteile $x_i$ aus dem aufgenommenen Spektrum sowie durch das Iterationsverfahren mit Hilfe der Vorgabewerte für Kompressibilitätszahl K und den Realgasfaktor $Z_n$ nach einer entsprechenden Anzahl von Iterationsschritten der tatsächlich in dem Probengas vorliegende Wert für die Kompressibilitätszahl K und daraus dann die Werte für Normbrennwert $H_{v,n}$ und Normdichte $\rho_n$ mit hinreichender Genauigkeit bestimmen. Hierbei wird die Kompressibilitätszahl K aus den spektroskopisch meßbaren Größen im Betriebszustand sowie den Startwerten für die Kompressibilitätszahl K und den Realgasfaktor $Z_n$ ermittelt und die Realgasberechnungen selbst anhand von Standard-Berechnungsverfahren vorgenommen werden und diese Berechnungsverfahren selbst in der Regel wiederum Iterationsverfahren sind. Es liegt also eine zweistufige Iteration vor, bei der in einem ersten Iterationsschritt mit den Vorgabewerten für Kompressibilitätszahl K und Realgasfaktor $Z_n$ und den Eingangsgrößen des Standard-Berechnungsverfahrens mittels Iterationsverfahren die Kompressibilitätszahl K berechnet wird und bei Nichtkonvergenz die Kompressibilitätszahl K und die daraus wiederum ermittelbaren Größen nach einer Neuberechnung der Eingangsgrößen des Standard-Berechnungsverfahrens wiederum als Eingangswerte für eine neue Iterationsschleife eingesetzt werden. Neben der Konvergenz der Kompressibilitätszahl K kann auch die Konvergenz von Normbrennwert $H_{v,n}$ und Normdichte $\rho_n$ überprüft werden. Diese Vorgehensweise hat den besonderen Vorteil, daß aus den im Betriebszustand meßbaren Größen des Probengases sowie dem aus dem Betriebszustand ermittelten Spektrum unmittelbar die Normwerte des Probengases bestimmt werden können, ohne daß das Probengas sonst notwendigen, aufwendigen Behandlungen unterzogen werden muß, um es auf Normbedingungen zu bringen.

In einer besonders bevorzugten Ausgestaltung wird als Standard-Berechnungsverfahren für die Bestimmung der Kompressibilitätszahl K das Iterationsverfahren AGA8-92DC genutzt, das als international einheitliches Berechnungsverfahren in der ISO 12213/2 festgelegt ist. Als wesentliche Eingangsgrößen für dieses Verfahren AGA8-92DC werden hier die molaren Stoffmengen der infrarotaktiven Komponenten sowie die molare Stoffmenge von Stickstoff $N_2$ benutzt.

In einer anderen vorteilhaften Ausgestaltung wird als Standard-Berechnungsverfahren das Iterationsverfahren GERG88 benutzt, das in dem DVGW-Arbeitsblatt 486 festgelegt und ausführlich beschrieben ist. Dieses Iterationsverfahren läßt ebenfalls die Bestimmung der Kompressibilitätszahl K zu, wobei als Eingangsgrößen aus den molaren Stoffmengen der infrarotaktiven Komponenten der Normbrennwert $H_{v,n}$, die Normdichte $\rho_n$ und die Konzentration von $CO_2$ berechnet werden kann.

In besonders vorteilhafter Ausgestaltung wird die Ermittlung der Stoffmengenanteile $x_i$ der infrarotaktiven Komponenten des Probengases im Betriebszustand aus dem aufgenommenen Spektrum mittels eines Verfahrens nach der sog. Multivariaten Analyse (MVA) vorgenommen, die in dem Bericht des Gas Research Institute GRI-93/0083 "Optical BTU Sensor Development" ausführlich beschrieben ist. Anhand dieses Verfahrens zur Multivariaten Analyse (MVA) werden aus unter Betriebsbedingungen aufgenommenen Spektren des Probengases die einzelnen Stoffmengenanteile der infrarotaktiven Bestandteile ermittelt und stehen daher für die Berechnung der Kompressibilitätszahl K zur Verfügung.

In vorteilhafter Ausgestaltung werden die Vorgabewerte für die Kompressibilitätszahl K und den Realgasfaktor $Z_n$ aus einem Kennfeld entnommen, das den Einfluß des Betriebsdruckes $p_b$ und der Betriebstemperatur $T_b$ für eine bekannte, dem Probengas ähnliche Gaszusammensetzung wiedergibt. Hierbei wird von der Tatsache Gebrauch gemacht, daß der Betriebsdruck $p_b$ einen großen Einfluß, die Betriebstemperatur $T_b$ sowie die tatsächliche Gaszusammensetzung, insbesondere für übliche Brenngaszusammensetzungen, nur geringen Einfluß auf die Werte für den Realgasfaktor $Z_n$ haben. Daher kann anhand von vorher einmal bestimmten Kennfeldern für dem Probengas ähnliche Gaszusammensetzungen, in denen die Abhängigkeit von Druck und Temperatur aufgetragen sind, mit guter erster Näherung für den im Probengas vorliegenden Betriebsdruck $p_b$ sowie die Betriebstemperatur $T_b$ ein Startwert für die iterative Berechnung nach dem Standard-Berechnungsverfahren entnommen werden, der mit recht guter Genauigkeit schon nach wenigen Iterationsschritten gegen die tatsächliche Kompressibilitätszahl K konvergiert.

Es ist weiterhin besonders vorteilhaft, daß direkt aus dem aufgenommenen Spektrum die Stoffmengen der einzelnen brennbaren Komponenten des Probengases im Betriebszustand und die Stoffmenge des Stickstoffes $N_2$ an dem Probengas als Funktion der Stoffmengen der infrarotaktiven Komponenten des Probengases ermittelt werden. Hierbei geht als zusätzliche Information außerdem der Druck des Probengases, der auch die Stoffmenge des Stickstoffes berücksichtigt, in die Berechnung ein.

In weiterer Ausgestaltung kann die Stoffmenge des Stickstoffes $N_2$ an dem Probengas sich mit den Stoffmengen der infrarotaktiven Komponenten des Probengases zum Gesamtvolumen des Probengases ergänzen, wobei weitere Stoffe, wie z.B. Sauerstoff $O_2$, Wasserstoff $H_2$ und Wasser z.B. in typischen Brenngasen nur in geringen Spuren auftreten und daher in der Regel vernachlässigt werden können.

Die Erfindung betrifft weiterhin ein Verfahren zur Bestimmung der Gasqualität eines Probengases, insbesondere eines Brenngases, ausgehend von einem unter Betriebsbedingungen mittels infrarotspektroskopischer Meßverfahren bestimmten Spektrums des Probengases.

Dieses Verfahren wird dadurch in erfindungsgemäßer Weise weiterentwickelt, daß in einem ersten Verfahrensschritt Vorgabewerte für Kompressibilitätszahl K und Realgasfaktor $Z_n$ zur Berechnung der gesuchten Kompressibilitätszahl K vorgegeben werden, in einem weiteren Verfahrensschritt aus dem in Zustand des Probengases unproblematisch ermittelbaren Betriebsdruck $p_b$ und der Betriebstemperatur $T_b$ des Probengases sowie dem Betriebsbrennwert $H_{v,b}$ und der Betriebsdichte $\rho_b$ Eingangsgrößen für die Bestimmung der Kompressibilitätszahl K berechnet werden, wobei als weitere Eingangsgröße die molare Stoffmenge von $CO_2$ aus einer weiteren Absorptionsbande des ermittelten Spektrums bestimmt wird. Hierbei werden sowohl der Betriebsbrennwert $H_{v,b}$ als auch die Betriebsdichte $\rho_b$ direkt aus

dem Spektrum bestimmt, wobei gleichzeitig aus dem gleichen Spektrum die weitere Absorptionsbande für die Stoffmenge von $CO_2$ ermittelt werden kann. Mit diesen Eingangsgrößen wird dann in einem weiteren Verfahrensschritt nach dem Iterationsverfahren GERG88 die Kompressibilitätszahl K berechnet. Anhand der Ergebnisse des Iterationsverfahrens GERG88 wird dann untersucht, ob der Wert für die Kompressibilitätszahl K konvergiert und dann daraus der volumetrische Normbrennwert $H_{v,n}$, die Normdichte $\rho_n$ und der Stoffmengenanteil $X_{CO_2}$ berechnet. Ist ein Konvergieren festzustellen, so können als Ergebnisse des erfindungsgemäßen Verfahrens die ermittelte Kompressibilitätszahl K sowie die daraus berechneten Werte für Normbrennwert $H_{v,n}$ und Normdichte $\rho_n$ und $X_{CO_2}$ direkt weiter verwendet werden. Ist ein Konvergieren noch nicht festzustellen, so wird eine iterative Neuberechnung der Eingangsgrößen des Standard-Berechnungsverfahrens mit dem zuvor ermittelten Wert der Kompressibilitätszahl K solange durchgeführt, bis ein Konvergieren der ermittelten Kompressibilitätszahl K und ggf. von Normbrennwert $H_{v,n}$ und Normdichte $\rho_n$ und $X_{CO_2}$ festzustellen ist. Bei jeder Iteration wird daher der gerade ermittelte Wert der Kompressibilitätszahl K wieder in die Gleichungen zur Berechnung der Eingangsgrößen des Standard-Berechnungsverfahrens eingesetzt und ein neuer Iterationsschritt durchgeführt. Das erfindungsgemäße Verfahren weist den Vorteil auf, daß auch ohne Kenntnis der genauen Stoffmengen der einzelnen in einem Probengas enthaltenen Bestandteile die gewünschten Zielgrößen im Normzustand allein aus den spektroskopischen Größen im Betriebszustand sowie den Startwerten für die Kompressibilitätszahl K und den Realgasfaktor $Z_n$ bestimmt werden können, wobei ein zweistufiger Iterationsansatz, einmal mit der Iterationsberechnung der Kompressibilitätszahl K innerhalb des Verfahrens GERG88, zum anderen mit einer Iteration unter Neuberechnung der Eingangsgrößen für GERG88 mit der im vorherigen Schritt ermittelten Kompressibilitätszahl K bis zum Konvergieren der Ergebnisse für die Kompressibilitätszahl K und ggf. den daraus ermittelbaren volumetrischen Normbrennwert $H_{v,n}$ und die Normdichte $\rho_n$ durchgeführt werden kann. Diese sogenannte direkte Spektralauswertung (DSA) ist für die Bestimmung des Betriebsbrennwertes $H_{v,b}$ aus dem Stand der Technik grundsätzlich bekannt, wobei die Berechnung der Betriebsdichte $\rho_b$ sowie der Kompressibilitätszahl K ebenfalls allein aus dem aufgenommenen Spektrum bisher nicht bekannt war.

Besonders vorteilhaft ist es, daß der Betriebsbrennwert $H_{v,b}$ und die Betriebsdichte $\rho_b$ mittels spektraler Gewichtungsfunktionen direkt aus dem ermittelten Spektrum des Probengases bestimmt werden können, wobei hierfür ausgenutzt wird, daß aus der Schwingungsfrequenz der Bindung der infrarotaktiven Gaskomponenten ihre spektrale Lage erkennbar ist und diese wiederum von der reduzierten Masse der Bindungspartner abhängt. Damit enthält das Spektrum in seinem Betrag und seiner spektralen Verteilung bei geeigneter Auswertung auch Informationen zur Bestimmung der Betriebsdichte $\rho_b$ des Gases.

In weiterer Ausgestaltung wird mit den spektralen Gewichtungsfunktionen der bewertete Einfluß der Stoffmengenanteile der einzelnen Komponenten des Probengases auf den Betriebsbrennwert $H_{v,b}$ und die Betriebsdichte $\rho_b$ wiedergegeben. Hierbei müssen aufgrund der spektralen Gewichtungsfunktionen jedoch nicht die einzelnen Stoffmengenanteile ausdrücklich bestimmt werden, sondern der Einfluß dieser Stoffmengenanteile ist in den spektralen Gewichtungsfunktionen repräsentiert.

Ebenfalls ist es denkbar, daß die Vorgabewerte für die Kompressibllitätszahl K und den Realgasfaktor $Z_n$ aus einem Kennfeld entnommen werden, das den Einfluß von Betriebsdruck $p_b$ und Betriebstemperatur $T_b$ für eine bekannte, dem Probengas ähnliche Gaszusammensetzung wiedergibt. Wie schon vorstehend für das Verfahren nach Anspruch 1 ausgeführt, lassen sich hiermit in erster Näherung recht gute Startwerte für die Kompressibilitätszahl K und den Realgasfaktor $Z_n$ ermitteln, die schnell zu einer Konvergenz auch dieses erfindungsgemäßen Verfahrens beitragen.

Die Erfindung betrifft weiterhin eine gattungsgemäße photometrische Vorrichtung zur Bestimmung des Transmissionsspektrums eines Probengases, insbesondere zur Durchführung eines der Verfahren nach Anspruch 1 bzw. Anspruch 8.

Eine derartige gattungsgemäße Vorrichtung wird dadurch weitergebildet, daß die spektrale Schalteinheit eine Chopperanordnung aufweist, die aufgrund ihres selektiven Durchlaßverhaltens nur bestimmte Spektralbereiche des durch das Probengas erzeugten Spektrums in der Meßstrahlung zu dem Strahlungsempfänger durchläßt. Hierbei weist die Chopperanordnung eine Blende mit einer spiralförmigen Öffnung auf, bei der die Freigabe der Wellenlängenbereiche der Meßstrahlung kontinuierlich über das ganze Spektrum erfolgt. Hierdurch können auch Zusammensetzungen von Probengasen bestimmt werden, deren Spektren über weite Bereiche erfaßt werden müssen, auch erreicht man hierdurch eine größere Flexibilität bei der Auswertung der Spektren.

Hierbei kann in weiterer Ausgestaltung die Chopperanordnung ein derartiges Durchlaßverhalten aufweisen, daß die durchgelassenen Spektralbereiche insbesondere zur weiteren Auswertung mittels Verfahren der direkten Spektralauswertung (DSA) geeignet sind. Durch die Chopperanordnung wird in vereinfachender Weise nur derjenige Teil des gesamten Spektrum ausgemessen, der für die Bestimmung der Gasqualität z. B. eines Probengases typischer Zusammensetzung überhaupt von Interesse ist und dabei mit einfachen mechanischen Mitteln eine genaue Selektion dieser Spektrenbereiche erreicht. Auch ist eine derartige Chopperanordnung einfach aufzubauen und mechanisch stabil und unempfindlich.

Von besonderem Vorteil ist es, wenn die durch die Chopperanordnung jeweils durchgelassene Wellenlänge der Meßstrahlung durch Erfassung der Drehstellung einer Blende ermittelbar ist. Dies kann auf einfache Welse z.B. durch einen Drehgeber realisiert werden, der mechanisch einfach aufgebaut, kostengünstig und robust ist und eine sichere Synchronisation der Spektrenerfassung mit der jeweiligen Wellenlänge erlaubt.

In einer weiteren denkbaren Ausgestaltung weist die Chopperanordnung zwei alternierend die Meßstrahlung freigebende Sektorelementgruppen auf, wobei ein erster optischer Wellenleiter die durch die Sektorelemente der ersten Sektorelementgruppe freigegebene Meßstrahlung in die Probenzelle und nach dem Durchgang durch die Probenzelle zu dem Strahlungsempfänger und ein zweiter optischer Wellenleiter die durch die Sektorelemente der zweiten Sektorelementgruppe freigegebene Meßstrahlung direkt zu dem Strahlungsempfänger leitet. Bei der hier vornehmlich zugrunde liegenden Anwendung werden sehr hohe Anforderungen an die Langzeitstabilität der Vorrichtung gegen Signaldrift gestellt. Die folgenden drei Faktoren tragen dabei zu einer Drift des Signals bei: Fluktuation in der Lichtleistung der Strahlungsquelle, Fluktuation der Empfindlichkeit des Strahlungsempfängers (beide z. B. infolge von Alterungsprozessen), sowie Fluktuation der $CO_2$-Konzentration in der Umgebungsluft, die ein instabiles $CO_2$-Untergrundspektrum zur Folge hat. Der $CO_2$-Gehalt in der Umgebungsluft und somit auch im offenen Strahlengang einer Vorrichtung variiert z.B. durch Anwesenheit von Personen in der Umgebung, Alle drei Einflüsse werden kompensiert durch den hier vorgeschlagenen Zweistrahl-Aufbau der Vorrichtung, bei dem ein ständiger Vergleich des Signals mit einem Referenzsignal erfolgen kann.

Durch die Sektorelemente der Sektorelementgruppen wird die jeweils freigegebene Meßstrahlung desjenigen optischen Wellenleiters, der direkt zu dem Strahlungsempfänger geleitet wird, als Referenz zum Eliminieren des Einflusses von in der Umgebung der Probenzelle und/oder der Vorrichtung vorhandenen $CO_2$ Veränderungen der Strahlungsquelle und/oder des Strahlungsempfängers genutzt. Neben der Elimination des Einflusses des $CO_2$ in der Umgebung können auf diese Weise auch Veränderungen der Strahlungsquelle und/oder des Strahlungsempfängers eliminiert werden, die sich z.B. durch Alterungserscheinungen oder Verschmutzungen einstellen können.

Hierbei kann in einer ersten Ausgestaltung die durch die Sektorelemente der Sektorelementgruppen jeweils freigegebene Meßstrahlung über ersten und zweiten optischen Wellenleiter in einem oder mehreren Filtern oder einem dispersiven Element, vorzugsweise einem Gitter-Monochromator, zusammengeführt sein.

Es ist von besonderem Vorteil, wenn der Strahlungsempfänger die aus einem oder mehreren Filtern oder dem dispersiven Element austretende, durch die Sektorelemente der Sektorelementgruppen jeweils freigegebene Meßstrahlung beider optischer Wellenleiter erfaßt.

In einer weiteren Ausgestaltung ist es denkbar, daß die durch die Sektorelemente der Sektorelementgruppen jeweils freigegebene Meßstrahlung über ersten und zweiten optischen Wellenleiter dem Eingang des einen oder der mehreren Filter oder dem dispersiven Element zugeführt sind, wobei an der Chopperanordnung ebenfalls vorhandene weitere Sektorelementgruppen die aus dem einen oder den mehreren Filtern odar dem dispersivem Element austretende Meßstrahlung alternierend auf den Strahlungsempfänger aufschalten. Hierdurch kann mit einer mechanisch einfach aufgebauten Anordnung der Sektorelementgruppen auf der Blende eine sichere Synchronisation der Modulationen sichergestellt werden. Hierbei ist eine weitere Variante dadurch denkbar, daß die Chopperanordnung sowohl die Auswahl der Wellenlängen für das Spektrum als auch das alternierende Umschalten der Meßstrecke zwischen den Wellenleitern vornimmt.

Ebenfalls ist es in weiterer Ausgestaltung denkbar, daß die durch die Sektorelemente der Sektorefementgruppen jeweils freigegebene Meßstrahlung über ersten und zweiten optischen Wellenleiter in einem Y-Faserkoppler zusammengeführt sind, der die Meßstrahlung des ersten und des zweiten optischen Wellenleiters dem einen oder den mehreren Filtern oder dem dispersiven Element zuführt. Dies erlaubt eine besonders einfache Gestaltung des Filters und des dispersiven Elements und der Zusammenführung der geteilten Strahlen.

In weiterer Ausgestaltung ist es auch denkbar, die Probenzelle mit einem infrarotinaktiven Gas, vorzugsweise z. B. Stickstoff $N_2$, spülbar ist, um eine Nullmessung zum Ausgleich von Verschmutzungen oder dgl. der optischen Einrichtungen der Vorrichtung durchzuführen. Hierbei kann mit dieser mit Gas definierter und bekannter Eigenschaften gefüllten Probenzelle erfaßt werden, ob Abweichungen zu dem Ausgangszustand vorliegen und diese Abweichungen für weitere Messungen dann entsprechend berücksichtigt werden.

Einen besonders bevorzugte Ausführungsform der erfindungsgemäßen Verfahren sowie der erfindungsgemäßen Vorrichtung zeigt die Zeichnung.

Es zeigen:

Figur 1 - eine typische Verteilung innerhalb eines Spektrums für verschiedene der in Erdgasen vorhandenen Bestandteile,

Figur 2a - Grundsätzlicher Ablauf der zweistufigen Iterationsverfahren gemäß Anspruch 1,

Figur 2b - Grundsätzlicher Ablauf des zweistufigen Iterationsverfahren gemäß Anspruch 8,

Figur 3 - einen grundsätzlichen Aufbau einer photometrischen Vorrichtung aus den Stand der Technik,

Figur 4 - eine Vorrichtung, die über Lichtwellenleiter an eine Probenzelle angekoppelt ist und anhand von Filtern die Spektren bestimmt,

Figur 5 - eine Vorrichtung, bei der zur Wellenlängenselektion eine Chopperanordnung mit einer Sektorelementblende vorgesehen ist,

Figur 6 - eine erfindungsgemäße Vorrichtung nach Anspruch 12, bei der zur Wellenlängenselektion eine Chopperanordnung mit einer Spiralblende vorgesehen ist,

Figur 7 - eine weitere erfindungsgemäße Vorrichtung nach Anspruch 12 nach dem Referenzstrahlprinzip,

Figur 8 - eine Ausgestaltung der Vorrichtung gemäß Figur 7 mit Synchronisation über die Chopperanordnung selbst.

Figur 9 - eine Ausbildung der Probenzelle als Hohlwellenleiter,

Figur 10 - eine Anbindung der Probenzelle an einen Wellenleiter mit Hilfe von GRIND-Linsen,

Figur 11 - eine Ausgestaltung der Erfindung mit einer Modulation der Meßstrahlung.

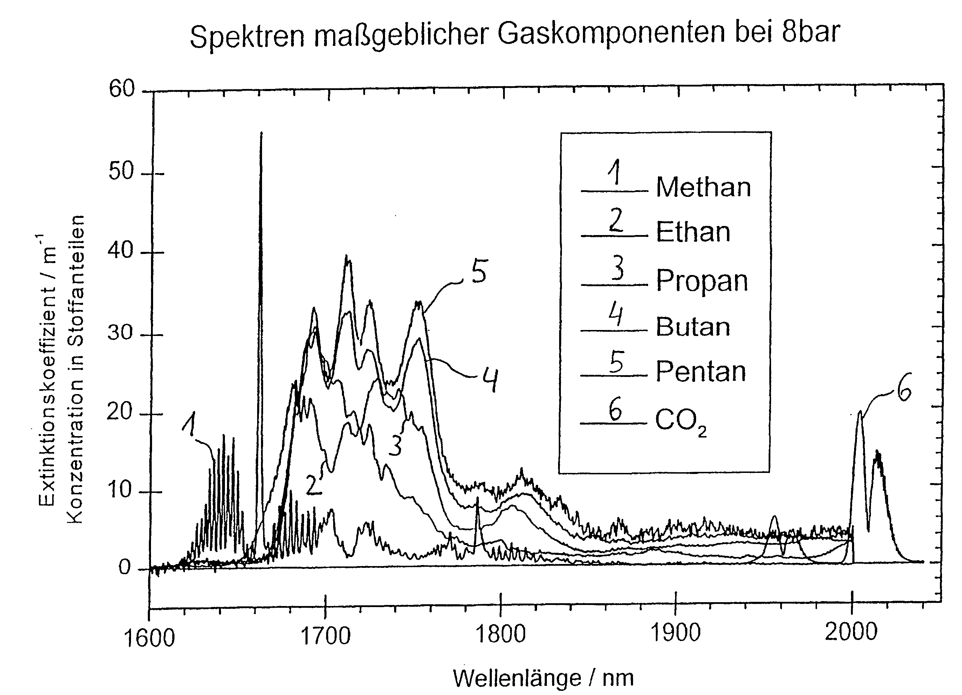

In der Figur 1 ist dargestellt, welche Verteilung sich innerhalb eines Spektrums für verschiedene der in Erdgasen vorhandenen Bestandteile ergibt.

Erdgas, wie es typischerweise in Europa verteilt wird, besteht im wesentlichen aus den Komponenten Methan, Ethan, Propan, Butan, Pentan und höheren Kohlenwasserstoffen sowie Kohlendioxid und Stickstoff. Nach DIN 51857 gelten als erdgasähnliche Gemische solche, deren Stoffmengenanteile die folgenden Bedingungen erfüllen:

$$X(CH4) >= 0.5$$
$$X(N2) <= 0.3$$
$$X(CO2) <= 0.15$$
$$X(C2H6) <= 0.15$$
$$\sum x_i \text{ über alle anderen Komponenten} <= 0.05$$

Tabelle 3: Grenzwerte für erdgasähnliche Gemische nach DIN 51587

Die restlichen Komponenten sind vor allem höhere Kohlenwasserstoffe mit Stoffmengenanteilen von etwa $10^{-3}$, weitere Stoffe wie $O_2$, $H_2$, Wasser und andere treten in den typischen Erdgasen in Spuren auf, die eine Messung mit der geforderten Genauigkeit nicht stören. Mit Ausnahme des Stickstoffs sind somit alle wesentlichen Komponenten erdgasähnlicher Gemische infrarotaktiv, d.h. sie absorbieren Strahlung im infraroten Spektralbereich und lassen sich so nachweisen.

Die Extinktionskoeffizienten der wesentlichen Gaskomponenten sind in Figur 1 dargestellt; während die Komponenten Methan und $CO_2$ in bestimmten Spektralbereichen isoliert stehen, überlappen sich die höheren Kohlenwasserstoffe spektral sehr stark. Die Figur 1 zeigt außerdem typische Spektralbereiche für die Auswertung der einzelnen Komponenten ($CH_4$, $CO_2$) bzw. Komponentengruppen ($C_2H_6$ und höhere Kohlenwasserstoffe).

Nach dem Beer-Lambert'schen Gesetz tragen die infrarotaktiven Komponenten zur Extinktion im Infrarotspektrum des Gases entsprechend ihrer Teilchendichte im optischen Meßvolumen bei. Aus dem Spektrum lassen sich somit für eine unbekannte Gasprobe die volumetrischen Stoffmengenkonzentrationen der infrarotaktiven Substanzen unter den Meß- bzw. Betriebsbedingungen ermitteln.

Zur Bestimmung des volumetrischen Brennwertes unter Betriebsbedingungen sind diese Informationen ausreichend, da der "unsichtbare" Stickstoff nicht zum Brennwert beiträgt. Nach dem Stand der Technik werden hierfür zwei Verfahren beschrieben: Bei dem ersten Verfahren (DVGW-Arbeitsblatt 486) erfolgt eine Analyse des Gasgemisches bezüglich seiner Zusammensetzung aus einzelnen Komponenten. Dabei werden aus den Spektren mittels des mathematischen Verfahrens der Multivariaten Analyse (MVA) die Stoffmengen der einzelnen brennbaren Komponenten bestimmt und der Betriebsbrennwert als entsprechend gewichtete Summe berechnet. Beim zweiten Verfahren (Direkte Spektralanalyse DSA) (ISO 12213-2:1997(E)) werden nicht einzelne Komponenten identifiziert, sondern durch Faltung mit einer speziellen Spektralfunktion wird der Betriebsbrennwert direkt aus dem Spektrum berechnet.

In der Praxis erwartet man von der Gasbeschaffenheitsmessung nicht nur den Brennwert $H_{v,b}$ unter Betriebsbedingungen, sondern den Brennwert $H_{v,n}$ unter Normbedingungen sowie die Normdichte $\rho_n$ und die Kompressibilitätszahl K. Zur Berechnung dieser Größen ist die Kenntnis des Stickstoffanteiles im Gas notwendig, dieser läßt sich aber aus dem Infrarotspektrum nicht direkt gewinnen.

Die hier vorgestellten Verfahren ermöglichen eine Bestimmung der maßgeblichen Gasgrößen aus dem Infrarotspektrum ohne explizite Kenntnis des Stickstoffanteils. Dabei wird der Stickstoffbeitrag als Funktion der anderen spektroskopisch meßbaren Komponenten ausgedrückt; als zusätzliche Information geht außerdem der Druck des Gases, der auch den Stickstoffanteil berücksichtigt, in die Darstellung mit ein. Grundlage ist dabei die Gasgleichung des realen Gases mit dem Druck p, dem Volumen V, dem Realgasfaktor Z als Funktion des Zustandes (p,T) und der Gaszusammensetzung $x_i$, der Summe aller Moleküle N, dem Boltzmannfaktor $k_B$ und der Temperatur T.

$$p\,V = Z(p,T,x_i)\,N\,k_B\,T$$

Damit ergeben sich die mathematischen Darstellungen der gesuchten Größen für ein erstes Verfahren nach Anspruch 1 zur Bestimmung der Kompressibilitätszahl K in den folgenden Gleichungen. Diese Darstellungen enthalten jedoch die gesuchte Kompressibilitätszahl K sowie den unbekannten Realgasfaktor $Z_n=Z(p_n, T_n)$ des unbekannten Gases unter Normbedingungen.

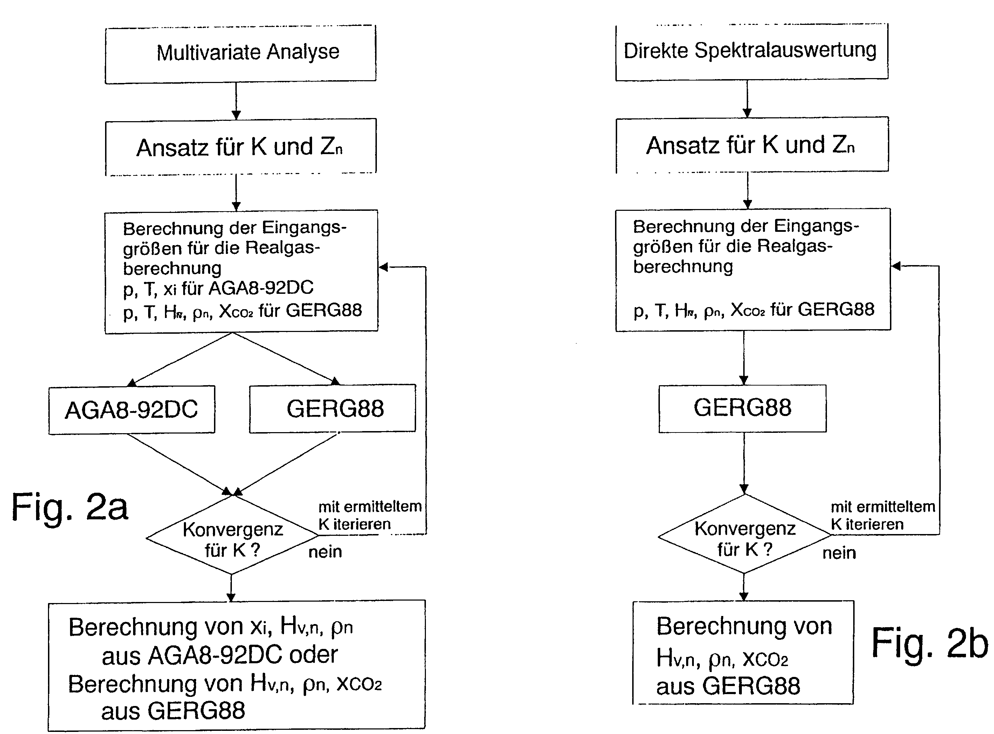

Die Berechnung erfolgt durch einen in Figur 2 näher erläuterten zweistufigen Iterationsansatz, bei dem geeignete Startwerte für K und $Z_n$ gewählt werden. Zur Bestimmung der Kompressibilitätszahl K wird entweder das Verfahren GERG88 (DVGW-Arbeitsblatt 486) oder das Verfahren AGA-92DC (ISO 12213-2:1997(E)) verwendet. Die entsprechenden Eingangswerte der beiden Iterationsverfahren werden aus der Spektralanalyse der Gase mit den geeignet gewählten Startwerten für $Z_n$ und K berechnet.

Die Eingangsgrößen für das Standard-Berechnungsverfahren AGA8-92DC werden mit den volumetrischen Stoffmengenkonzentrationen der infrarotaktiven Komponenten und den Startwerten für K und $Z_n$ wie folgt dargestellt:

$$X_i = \frac{K_b T_b}{p_b V_n} K Z_n N_i$$

$$x_{N2} = 1 - \frac{k_b T_b}{p_b V_b} K Z_n \sum_i N_i$$

Dabei bezeichnen die $x_i$ die Stoffmengenanteile der infrarotaktiven Komponenten und $X_{N2}$ den Stoffmengenanteil von Stickstoff.

Die Eingangsgrößen für das alternative Standard-Berechnungsverfahren GERG88 werden aus den volumetrischen Stoffmengenkonzentrationen der infrarotaktiven Komponenten berechnet und mit den Startwerten für K und $Z_n$ wie folgt dargestellt:

$$H_{v,n} = \frac{p_n T_b}{p_b T_n} K \sum_i H_{m,i} N_i$$

$$\rho_n = \frac{p_n T_b}{V_b p_b T_n} K \Sigma (m_i - m_{N_2}) N_i + \frac{p_n m_{N_2}}{T_n k_B Z_n}$$

$$X_{CO_2} = \frac{k_B T_b}{p_b V_b} K \, Z_n \, N_{CO_2}$$

mit Brennwerten $H_{m,i}$ pro Molekül der Komponente i und den entsprechenden Molekülmassen $m_i$, der Molekülmasse von Stickstoff $m_{N_2}$ und der Zahl der $CO_2$-Moleküle $NCO_2$ im Meßvolumen.

Eine Multivariate Analyse (MVA), wie sie in der Literatur (DVGW-Arbeitsblatt 486) beschrieben wird, liefert die volumetrischen Stoffmengen der Gaskomponenten im Betriebszustand. Das hier vorgestellte Verfahren nach Anspruch 1 beschreibt, wie außerdem mit diesen Daten der Normbrennwert $H_{v,n}$, die Normdichte $\rho_n$ sowie die Kompressibilitätszahl K ermittelt werden können.

Nach dem Verfahren nach Anspruch 8 lassen sich dagegen mittels spektraler Gewichtungsfunktion die Spektren direkt ohne detaillierte Auflösung der einzelnen Gaskomponenten auswerten. Die Bestimmung des Betriebsbrennwertes $H_{v,b}$ nach diesem Verfahren wird in der Patentanmeldung 198 38 301.0 beschrieben. Die volumetrische Stoffmenge von $CO_2$ unter Betriebsbedingungen kann als separate Absorptionsbande nach üblichen spektroskopischen Verfahren ausgewertet werden.

Ein wesentlicher Bestandteil des hier vorgestellten Verfahrens nach Anspruch 8 ist die spektrale Messung der Betriebsdichte $\rho_b$, dabei kommt ebenso die Faltung des Spektrums mit einer Spektralfunktion zum Einsatz. Der physikalische Hintergrund des Verfahrens hierbei ist, daß jede Bindung der infrarotaktiven Gaskomponente zur Extinktion beiträgt und so die Masse der Bindungspartner im Spektrum repräsentiert. Die Schwingungsfrequenz der Bindung und damit ihre spektrale Lage hängt von der reduzierten Masse der Bindungspartner ab. Damit enthält das Spektrum in seinem Betrag und seiner spektralen Verteilung bei geeigneter Auswertung Informationen zur Bestimmung der Dichte des Gases.

Die Eingangsgrößen für das hier verwendete Standard-Berechnungsverfahren GERG88 ergeben sich dabei aus der direkten Auswertung der Spektren und mit den Startwerten für K und $Z_n$:

$$H_{v,n} = \frac{p_n T_b}{p_b T_n} K \, H_{v,b}$$

$$\rho_{v,n} = \frac{p_n T_b}{p_b T_n} K \, \rho_{v,b}$$

$$X_{CO_2} = \frac{k_B T_b}{p_b V_b} K \, Z_n \, N_{CO_2}$$

In den Figuren 2a und 2b sind die Abläufe des zweistufigen Iterationsverfahrens gemäß den vorstehenden Gleichungen noch einmal genauer veranschaulicht. In der Figur 2a ist der prinzipielle Ablauf der Berechnung nach dem Verfahren gemäß Anspruch 1 mit den beiden alternativen Iterationsverfahren AGA8-92DC und GERG88 dargestellt, die von den Ergebnissen der Multivariaten Analyse (MVA) anhand des ermittelten Spektrums ausgehen. In der Figur 2b ist hingegen der prinzipielle Ablauf der Berechnung nach dem Verfahren gemäß Anspruch 8 dargestellt, das von den Ergebnissen der Direkten Spektralauswertung (DAS) ausgeht und dann das iterationsverfahren GERG88 nutzt.

Bei den Verfahren gemäß Figur 2a wird nach der Bestimmung der direkt mit üblichen Mitteln meßbaren Größen des Probengases wie Druck $p_b$ und Temperatur $T_b$ im Betriebszustand sowie der Auswertung des Spektrums mittels der Multivariaten Analyse (MVA) ein erster Ansatz für die Werte von K und $Z_n$ gemacht, der am einfachsten aus einem Kennfeld entnommen werden kann, das den Einfluß von Betriebsdruck $p_b$ und Betriebstemperatur $T_b$ für eine bekannte, dem Probengas ähnliche Gaszusammensetzung wiedergibt. Mit diesen Größen werden dann erstmalig je nach gewähltem Standard-Berechnungsverfahren (AGA8-92DC oder GERG88) die entsprechenden Eingangsgrößen des jeweiligen Standard-Berechnungsverfahrens berechnet und diese dann in dem jeweiligen Iterationsverfahren des Standard-Berechnungsverfahrens benutzt. Als Ergebnis ergibt sich ein Wert für die Kompressibilitätszahl K sowie die sich daraus berechnenden Größen Normbrennwert $H_{v,n}$ und Normdichte $\rho_n$. Konvergieren die Kompressibilitätszahl K, ggf. auch Normbrennwert $H_{v,n}$ und Normdichte $\rho_n$, gegen einen Endwert, so ist die Berechnung der gesuchten Größen beendet, denn diese liegen nun unmittelbar vor bzw. können anhand des berechneten Wertes für die Kompressibilitätszahl K ihrerseits berechnet werden. Kann man hingegen keine Konvergenz feststellen, so wird das Ergebnis der Kompressibilitätszahl K wieder in die Eingangsgrößen des Standard-Berechnungsverfahrens eingesetzt und mit diesen geänderten Eingangsgrößen die Iteration erneut gestartet und nach Durchlaufen der Verfahrensschritte erneut auf Konvergieren untersucht. Hierbei kann neben der Konvergenz des Wertes der Kompressibilitätszahl K, wie dies in den Figuren 2a und 2b dargestellt ist, zusätzlich auch die Konvergenz der aus der Kompressibilitätszahl K ermittelbaren Größen, z.B. Normbrennwert $H_{v,n}$ und Normdichte $\rho_n$ untersucht und als weiteres Abbruchkriterium der Iteration genutzt werden.

Bei dem Verfahren gemäß Figur 2b wird nach der Bestimmung der direkt mit üblichen Mitteln meßbaren Größen des Probengases wie Druck $p_b$ und Temperatur $T_b$ im Betriebszustand sowie der Auswertung des Spektrums mittels der Direkten Spektralauswertung (DAS) ein erster Ansatz für die Werte von K und $Z_n$ gemacht, der am einfachsten aus einem Kennfeld entnommen werden kann, das den Einfluß von Betriebsdruck $p_b$ und Betriebstemperatur $T_b$ für eine bekannte, dem Probengas ähnliche Gaszusammensetzung wiedergibt. Mit diesen Größen werden dann erstmalig nach Standard-Berechnungsverfahren GERG88 die entsprechenden Eingangsgrößen des Standard-Berechnungsverfahren berechnet und in dem Iterationsverfahren benutzt. Als Ergebnis ergibt sich ein Wert für die Kompressibilitätszahl K sowie die sich daraus berechnenden Größen Normbrennwert $H_{v,n}$ und Normdichte $\rho_n$. Konvergieren die Kompressibilitätszahl K, ggf. auch Normbrennwert $H_{v,n}$ und Normdichte $\rho_n$, gegen einen Endwert, so ist die Berechnung der gesuchten Größen beendet, denn diese liegen nun unmittelbar vor bzw. können anhand des berechneten Wertes für die Kompressibilitätszahl K ihrerseits berechnet werden. Kann man hingegen keine Konvergenz feststellen, so wird das Ergebnis der Kompressibilitätszahl K wieder in die Eingangsgrößen des Standard-Berechnungsverfahrens GERG88 eingesetzt und mit diesen geänderten Eingangsgrößen die Iteration erneut gestartet und nach Durchlaufen der Verfahrensschritte erneut auf Konvergieren untersucht. Hierbei kann neben der Konvergenz des Wertes der Kompressibilitätszahl K, wie dies in den Figuren 2a und 2b dargestellt ist, zusätzlich auch die Konvergenz der aus der Kompressibilitätszahl K ermittelbaren Größen, z.B. Normbrennwert $H_{v,n}$ und Normdichte $\rho_n$ untersucht und als weiteres Abbruchkriterium der Iteration genutzt werden.

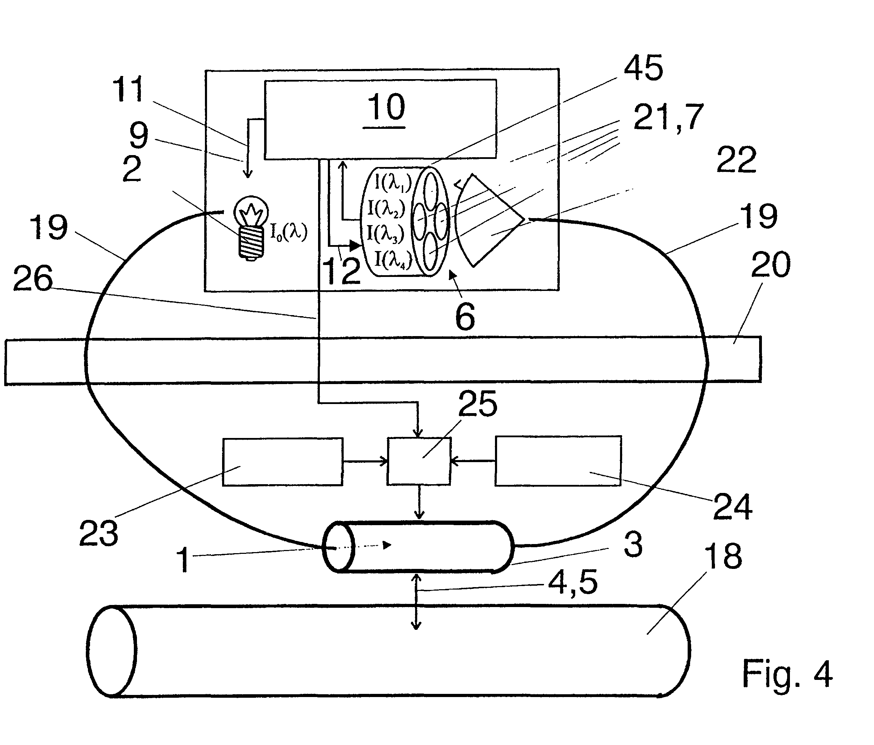

In den folgenden Figuren 4 bis 10 sind nun vorteilhafte Ausgestaltungen der erfindungsgemäßen Vorrichtung nach Anspruch 12 beschrieben. Gleiche Sachnummern bezeichnen jeweils identische oder funktionsähnliche Einrichtungen, so daß in den Figuren 4 bis 10 im wesentlichen nur noch die Unterschiede in den jeweiligen Ausführungsformen dargestellt werden und ansonsten auf die jeweils vorstehende Beschreibung verwiesen sei.

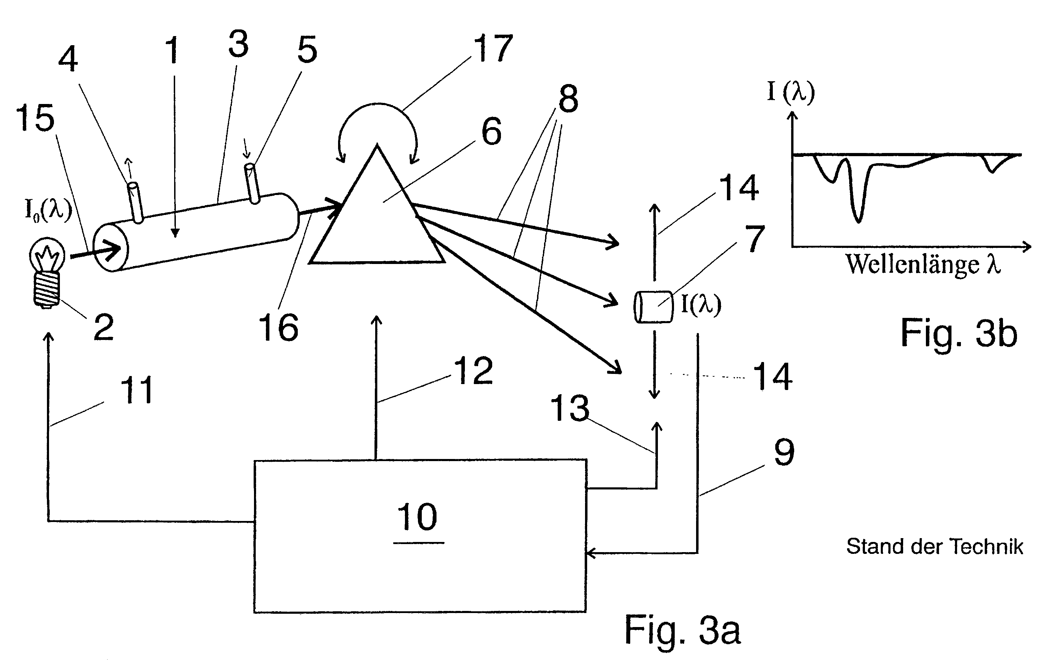

Die in der Figur 3a dargestellte photometrische Vorrichtung nach dem Stand der Technik weist eine Strahlungsquelle 2 auf, die spektral breitbandige Strahlung 15 im maßgeblichen Spektralbereich, hier vornehmlich im Infrarot emittiert. Die Strahlung 15 wird durch das Probengas 1 geführt, das sich in einer mittels der Öffnungen 4, 5 beschickbaren Probenzelle 3, zum Beispiel in einer optischen Meßküvette befindet. Die Strahlung 15 wird von dem Probengas 1 entsprechend seiner Stoffzusammensetzung wellenlängenselektiv absorbiert und die transmittierte Strahlung 16 einer Modulationseinheit 6 zugeführt, das eine spektrale Analyse der transmittierten Strahlung 16 durchführt. Hierzu kann, wie in der Figur 3 schematisch angedeutet, ein Prisma verwendet werden, bei dem die Strahlung 8 der verschiedenen Wellenlängen in unterschiedlichen Richtungen austritt. Auch sind Gitteranordnungen oder dgl. üblich. Die selektive Detektion einer bestimmten Wellenlänge erfolgt durch Positionierung eines strahlungsempfindlichen Strahlungsempfängers 7 entlang der Verstellrichtung 14 in der entsprechenden Position der spektral aufgeweiteten Meßstrahlung 8 oder durch Drehung des Prismas. Eine Rechnereinheit 10 zeichnet die mittels Signalen 9 übermittelte, detektierte Meßstrahlung 8 als Funktion der Wellenlänge auf und gewinnt so das Transmissionsspektrum gemäß Figur 3b des Probengases 1, das insbesondere nach den oben beschriebenen Verfahren bezüglich der gesuchten Gasgrößen ausgewertet werden kann. Neben der Signalverarbeitung dient die Rechnereinheit 10 außerdem zur Steuerung der Strahlungsquelle 2 mittels Signalen 11, z.B. zur Modulation der Lichtstärke, sowie zur Steuerung der Wellenlängenselektion mittels Signalen 12 über die Prismenbewegung 17 oder mittels Signalen 13 über die Bewegung des Strahlungsempfängers. Die Vorrichtung gemäß Figur 3a liefert ein Transmissionsspektrum, wie beispielhaft in Figur 3b dargestellt, das nach den oben beschriebenen Verfahren durch MVA oder durch DSA ausgewertet und mittels der oben beschriebenen Iterationsverfahren zu den gewünschten Normgrößen umgewertet wird.

In einer ersten denkbaren Ausführungsform nach Figur 4 ist die Vorrichtung über Lichtwellenleiter 19 an die Probenzelle 3 angekoppelt und kann so im ex-freien Raum hinter einer Ex-Barriere 20 installiert werden, während sich die eigentliche Meßzelle nahe der Gasleitung 18 im Ex-Raum befindet. Zur Aufnahme eines Leerspektrums und somit der Offset-Transmission des optischen Systemes kann die Probenzelle 3 mit einem spektroskopisch inaktiven Inertgas 23, im Infrarot z. B. Stickstoff gefüllt werden. Alternativ kann die Probenzelle 3 außerdem mit einem Kalibriergas 24 zur Überprüfung der Meßwerte beaufschlagt werden. Leer- und Kalibriermessung erfolgen in geeignet gewählten Zeitintervallen und werden über einen Ventilblock 25 von der Rechnereinheit 10 gesteuert. Diese Ausführungsform kann ebenso mit den anderen Ausführungen kombiniert werden.

Die Vorrichtung zeichnet gemäß Figur 4 kein spektral kontinuierlich aufgelöstes Spektrum auf, sondern verwendet zur Wellenlängenselektion optische Filter 21 (z. B. Interferenzfilter), die vor je einem Strahlungsempfänger 7 sitzen. Alternativ kann auch ein einzelner Strahlungsempfänger 7 verwendet werden, wobei die Filter 21 sequentiell z. B. durch ein Filterrad 45 in den Strahlengang einer Optik 22 gebracht werden. Die Filterbereiche werden z.B. so gewählt, daß sie die Spektralfunktion im oben beschriebenen DSA-Verfahren nachbilden und direkt die gesuchten Betriebsgrößen liefern. Die Umrechnung auf die Normgrößen erfolgt mittels der oben beschriebenen zweistufigen Iterationsverfahren. Eine besonders einfache Filterbestükkung bestünde z.B. aus vier Filtern: je einer für $CH_4$, höhere CH, $CO_2$ und Referenzgas.

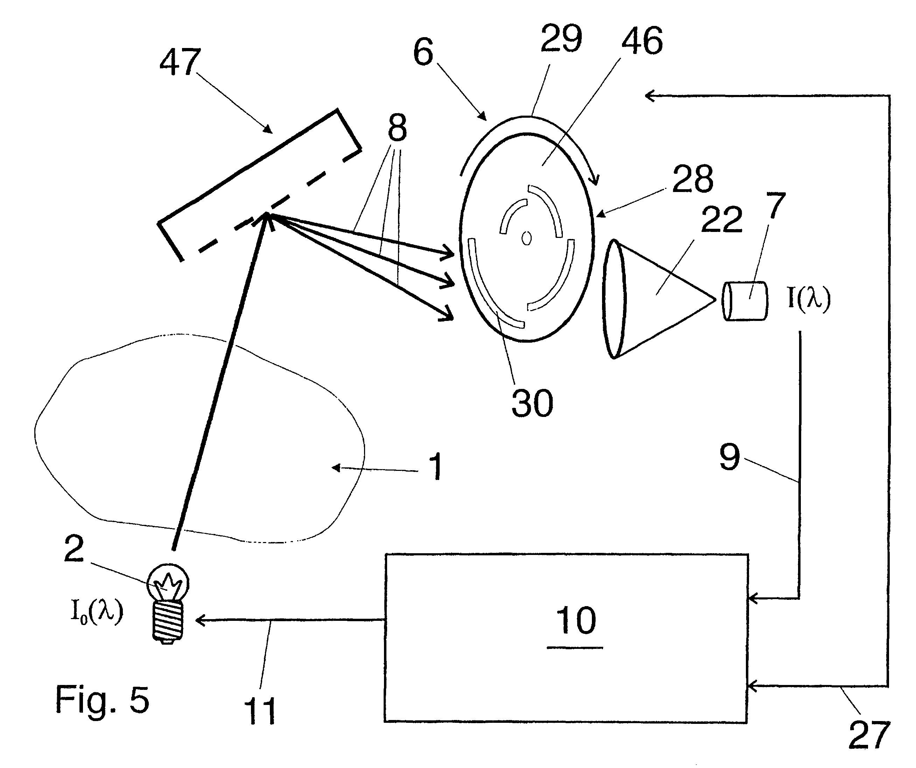

Die in der Figur 5 dargestellte Vorrichtung arbeitet in Kombination mit einem Prismen- oder Gitter-Spektrometer, wie auch in der Ausführungsform nach dem Stand der Technik gemäß Figur 3 vorgesehen. Die Wellenlängenselektion erfolgt dabei nicht durch Bewegung eines Prismas oder Gitters oder des Strahlungsempfängers 7, sondern durch eine in einer Chopperanordnung 28 vorgesehene Sektorblende 46. Diese Sektorblende 46 besitzt freie Sektoren 30, die sequentiell ausgesuchte Bereiche des Austrittsspaltes des Gitters 47 oder dgl. und damit ausgesuchte Wellenlängenbereiche des Spektrums der Meßstrahlung 8 freigeben. Eine Abbildungsoptik 22 bildet die transmittierte Meßstrahlung 8 auf einen Strahlungsempfänger 7 ab; aufgrund der spaltförmigen Geometrie bietet sich für die Abbildungsoptik 22 z.B. eine Zylinderlinse an. Mit der Information der Position der um die Drehrichtung 29 rotierenden Blende 46 und damit der Wellenlänge ermittelt die Rechnereinheit 10 ein vereinfachtes Transmissionsspektrum, das mit den oben beschriebenen Verfahren ausgewertet wird. Durch die Wahl der Lage und Breite der Sektoren 30 können Wellenlängenbereiche selektiert werden, die für die oben beschriebenen Auswerteverfahren, insbesondere für DSA, optimiert sind. Die Rechnereinheit 10 steuert mit Signalen 27 ebenfalls die Drehbewegung der Blende 46.

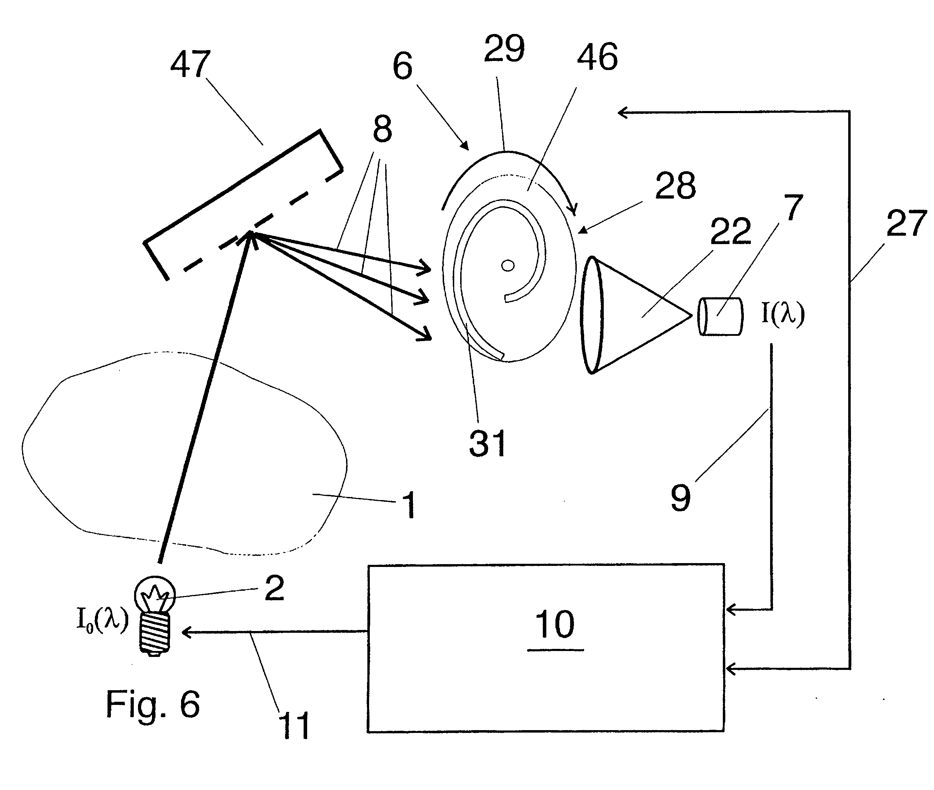

Eine erfindungsgemäße Bauform der Chopperanordnung 28 nach Anspruch 12 ist in der Figur 6 dargestellt. Die Chopperanordnung 28 arbeitet entsprechend zur Sektorblende 46 der Figur 5, wobei jedoch nicht sequentiell feste Wellenlängenintervalle eingeblendet werden, sondern das Spektrum vielmehr kontinuierlich durch eine spiralförmig in der Blende 46 angeordnete Spaltöffnung 31 abgetastet wird. Der Vorteil ist dabei eine höhere Flexibilität bei der Auswertung der Spektren, allerdings ist die Meßzeit für jedes einzelne Wellenlängenintervall verkürzt und somit das Signal-Rauschen erhöht. Wie bei der Chopperanordnung 28 nach Figur 5 ermittelt die Rechnereinheit 10 aus der Position der Blende 46 die jeweilig vorliegende Wellenlänge und nimmt somit ein Transmissionsspektrum für die obigen Auswerteverfahren MVA und DSA auf.

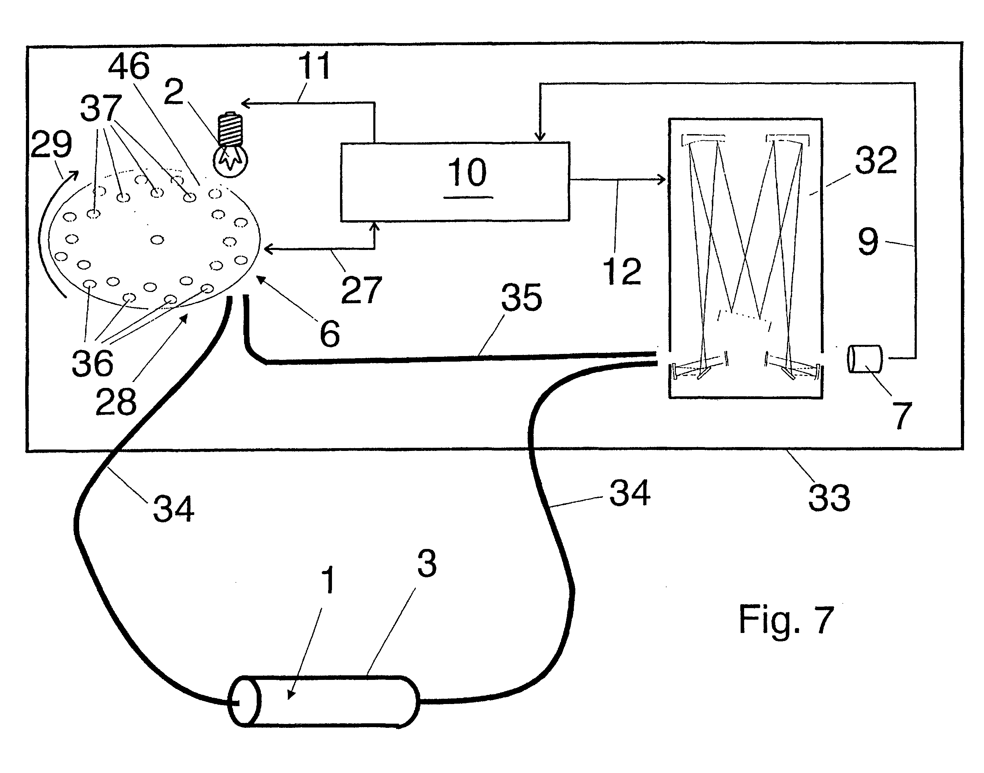

In der Figur 7 ist eine erweiterte Vorrichtung mit einer Chopperanordnung 28 dargestellt, bei der ein Referenzkanal durch einen optischen Wellenleiter 35 realisiert wird, der dafür sorgt, daß der offen in Umgebungsluft geführte optische Weg der Signale durch den Wellenleiter 34 und derjenige des Referenzstrahls durch den Wellenleiter 35 identisch sind, so daß z. B. das Untergrundspektrum des atmosphärischen $CO_2$ sowie Veränderungen von Strahlungsquelle und Strahlungsempfänger ideal unterdrückt werden kann. Der Wechsel zwischen den beiden Kanälen in den Wellenleitern 34, 35 erfolgt dabei durch eine entsprechende Chopperanordnung 28 zwischen Strahlungsquelle 7 und Einkopplung der Meßstrahlung 8 in die Wellenleiter 34, 35, der über unterschiedliche Sektorelementgruppen 36, 37 alternierend die Faserfacetten der beiden Wellenleiter beleuchtet. Die Enden der beiden Kanäle der Wellenleiter 34, 35 sind dicht übereinander im Eintrittsspalt des Monochromators 32 angeordnet, so daß beide Faserfacetten den gleichen Wellenlängenbereich abdecken und eine Verbreiterung des Spaltes und damit eine Verringerung der spektralen Auflösung nicht erforderlich ist. Die Abbildungen der beiden Faserfacetten im Austrittsspalt des Monochromators 32 liegen entsprechend ebenso übereinander und müssen im Strahlungsempfänger 7 vereinigt werden; dies erfolgt z. B. durch Wahl eines Strahlungsempfängers 7 mit genügend großer Fläche, um beide Lichtflecken zu erfassen, oder durch eine leicht defokussierte Justage des Strahlungsempfängers 7. Die eigentliche Meßvorrichtung kann hierbei z. B. in einem Gehäuse 33 gekapselt gegenüber Umgebungsbedingungen eingebaut sein.

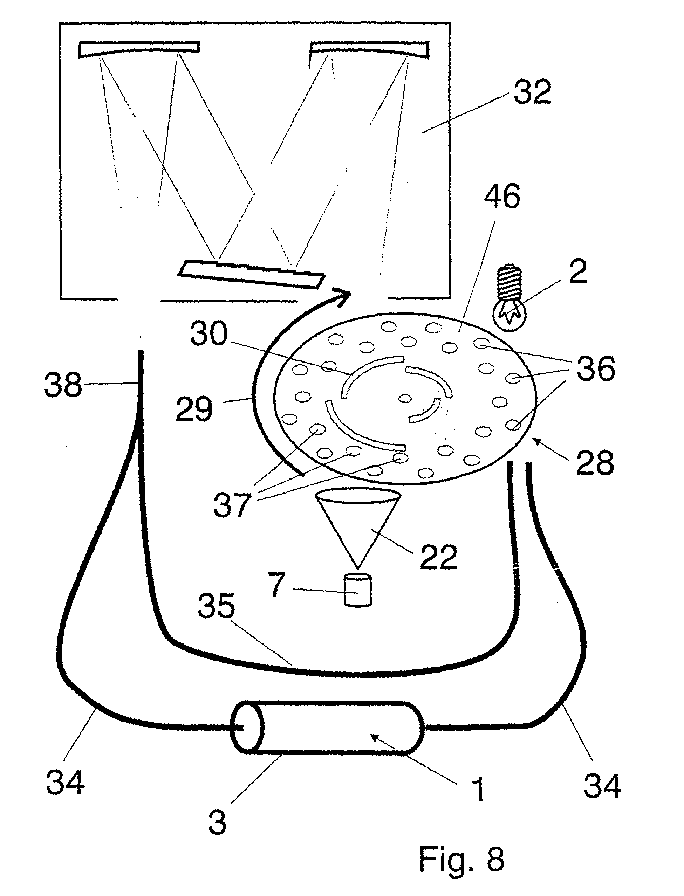

Eine weitere Variante der erfindungsgemäßen Vorrichtung zeigt die Figur 8. Bei dieser Ausführung übernimmt die Chopperanordnung 28 gleichzeitig zwei Funktionen: zum einen wird entsprechend den Ausführungen nach den Figuren 6 und 7 die Wellenlängenselektion mittels Segmentöffnungen 30 durchgeführt, zum anderen erfolgt die Umschaltung zwischen Signal- und Referenzkanal nach Figur 7 über entsprechende Segmentöffnungen 36, 37 im Randbereich derselben Blende 46. Der Vorteil dieser Ausführung ist die kompakte und einfache Ausführung beider Funktionen mit nur einem Antrieb, außerdem ist automatisch die Synchronität der Wellenlängenmodulation mit der Modulation des Strahlenkanals gewährleistet. In der Darstellung der Figur 8 wird außerdem ein sogenannter Y-Faserkoppler 38 verwendet, um die Strahlung von Referenzkanal und Signalkanal im Eingangsspalt des Monochromators 32 zu vereinen.

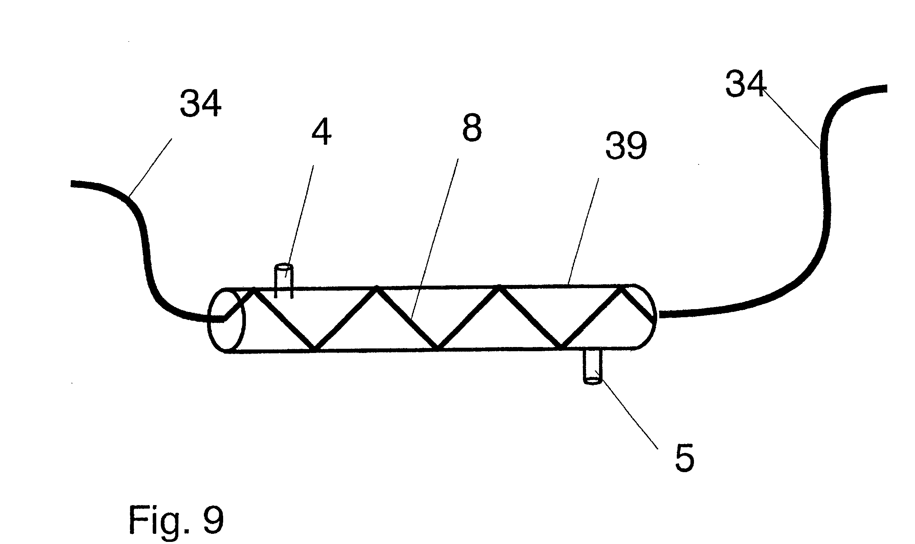

Die Figur 9 zeigt eine Ausbildung einer Probenzelle 3 in Form eines Hohlwellenleiters 39. Bei optischen Probenzellen 3 mit Ankopplung eines Wellenleiters 34 wird üblicherweise die Meßstrahlung 8, die aus dem Wellenleiter 34 austritt, durch eine Optik kollimiert und als Parallelstrahl durch die Probenzelle 3 geführt, um anschließend mit einer weiteren Optik wieder in den Wellenleiter 34 fokussiert zu werden. In Figur 9 ist die Probenzelle 3 als optischer Hohlwellenleiter 39 realisiert. Ein optischer Hohlwellenleiter 39 ist im allgemeinen ein Röhrchen mit rundem Querschnitt und einer innen verspiegelten Wand, so daß die eingekoppelte Meßstrahlung 8 durch Vielfachreflektion durch den Hohlwellenleiter 39 geführt wird. Die Dimensionierung des Querschnittes des Hohlwellenleiters 39 liegt vorzugsweise etwa in der Größenordnung des Wellenleiters 34. Hierdurch werden zusätzliche Optiken zur Ankopplung von Wellenleiter 34 und Hohlwellenleiter 39 unnötig.

Die Ausführung der Probenzelle 3 als Hohlwellenleiter 39 hat dabei folgende Vorteile: Die Probenzelle 3 läßt sich als Hohlwellenleiter 39 sehr einfach und ohne zusätzliche Optiken an den zu- und abführenden Wellenleiter 34 ankoppeln. Der Aufbau ist wesentlich kompakter als bei einem frei geführten Parallelstrahl. Die effektive Weglänge

**EP 1 141 677 B1**

der Meßstrecke und damit auch die Empfindlichkeit wird durch den Zick-Zack-Pfad erhöht. Da der Hohlwellenleiter 39 als Rohr ausgeführt ist, läßt er sich außerdem über Anschlüsse 4, 5 gut mit Gasleitungen 18 verbinden.

Eine weitere Anwendung dieser optischen Probenzelle 3 in Form eines Hohlwellenleiters 39 kann die optische Detektion für Gaschromatographen sein. Der Gasstrom, der aus der Kapillarsäule des Gaschromatographen austritt wird, direkt durch den Hohlwellenleiter 39 geführt und optisch vermessen.

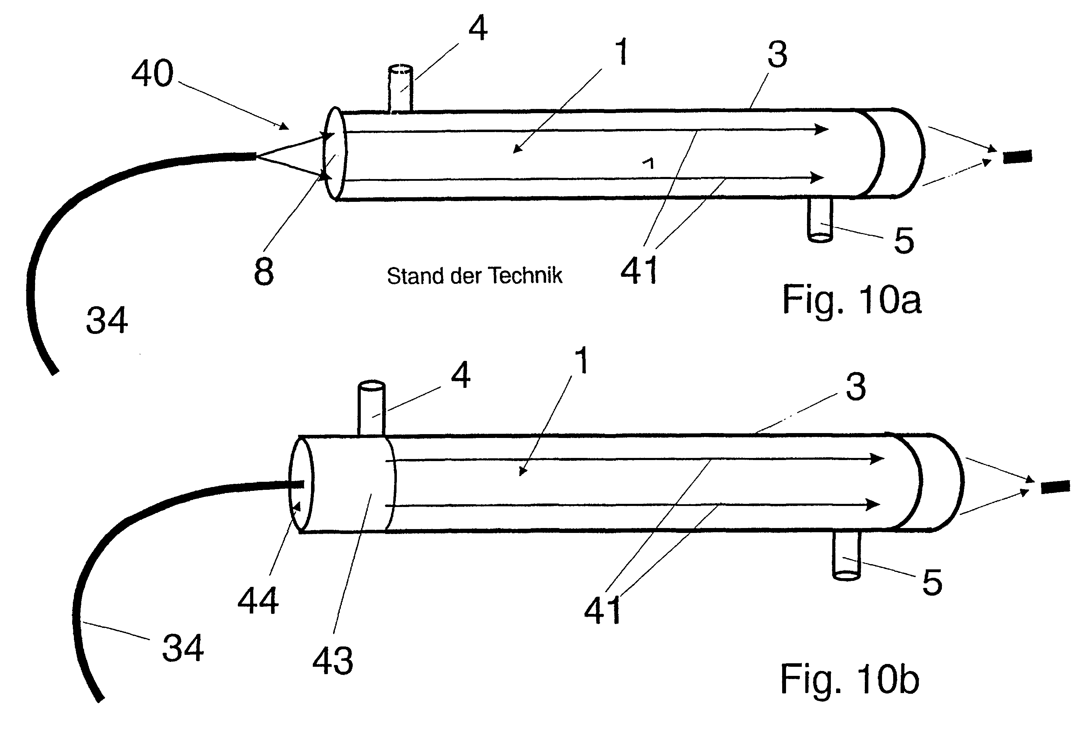

Die Figur 10b zeigt eine weitere Verbesserung der Ankopplung des Wellenleiters 34 an die Probenzelle 3 mittels sog. GRIND-Linsen 43. In der atmosphärischen Luft sind Gase wie $CO_2$ und Wasserdampf enthalten, die durch ihre Fluktuation die Gasmessung in der Probenzelle 3 aus dem Stand der Technik gemäß Figur 10a stören könnten; es muß daher vermieden werden, daß die Meßstrahlung 8 durch freie optische Wege 40 mit Umgebungsluft geführt wird. Im Spektrometer wird dieser Effekt durch ein Zweistrahlkonzept kompensiert; in der Probenzelle 3 treten jedoch ebenso freie Wegstrecken 40 in der Faseroptik auf. Durch Verwendung von Linsen mit einem radialen Brechzahlgradienten, sogenannten GRIND-Linsen 43 gemäß Figur 10b kann die Kollimation und Fokussierung ohne freie optische Wege 40 erfolgen und so der Einfluß der Messung durch atmosphärische Luft unterdrückt werden.

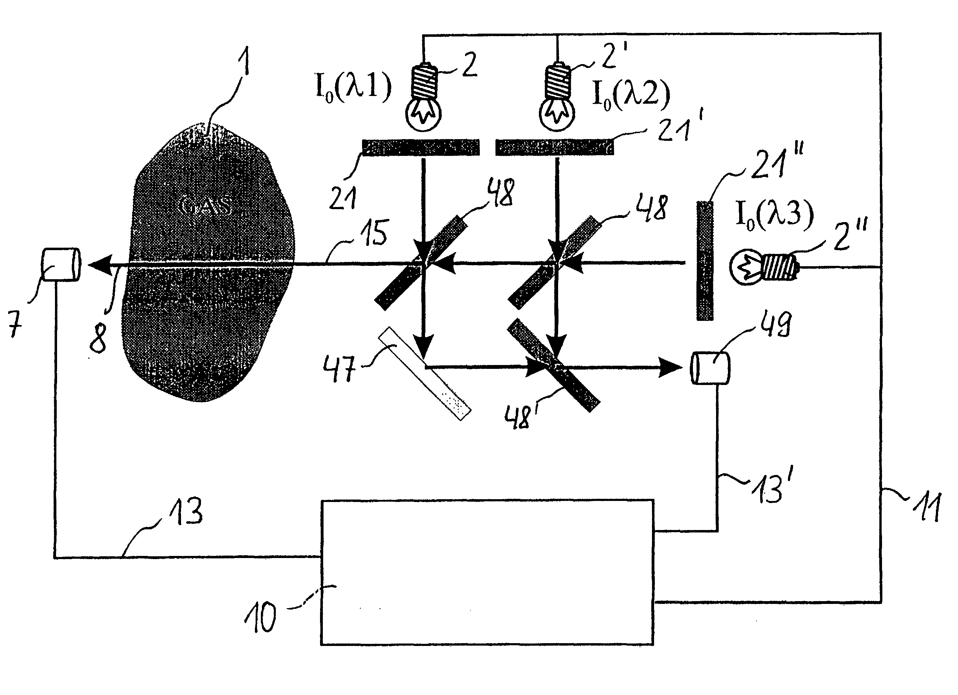

In der Figur 11 ist eine Ausgestaltung der Erfindung mit Modulation der Meßstrahlung dargestellt, bei der jeweils auf bestimmte Spektralbereiche der digitalen Signalauswertung ausgelegte Filter 21, 21' und 21" zugeordnete Strahlungsquellen 2, 2' und 2" benachbart angeordnet sind und die Strahlung 15 vor Durchtreten des Probengases 1 durch eine Anordnung von Strahlungsteilern 48 der Strahlung 15 vereinigt werden. Hierbei sind die Filter 21, 21' und 21" in ihrem Filterverhalten so ausgelegt sind, daß sie nur Spektralbereiche der von den Strahlungsquellen 2, 2' und 2" emittierten Strahlung durchlassen, die zu den gewünschten Spektralbereichen für die digitale Signalauswertung gewünscht werden. Nach der Strahlvereinigung dieser drei Teilstrahlen mit Hilfe der Strahlungsteiler 48 durchtritt die Strahlung 15 eine nicht weiter dargestellte Probenzelle 3, in der Probengas 1 enthalten ist. Die nach dem Durchtreten der nicht dargestellten Probenzelle 3 dort austretende Meßstrahlung 8 wird in schon grundsätzlich bekannter Weise von einem Strahlungsempfänger 7 aufgefangen und in ebenfalls schon dargestellter Weise an die Rechnereinheit 10 gemeldet. Diese Rechnereinheit 10 steuert hierbei sowohl den Strahlungsempfänger 7 sowohl mittels Steuersignalen 13 als auch die Strahlungsquellen 2, 2' und 2" mittels der Steuersignale 11, wobei sowohl die Strahlungsquellen 2, 2' und 2" derart veränderbar sind, daß eine Modulation der Strahlungsquellen 2, 2' und 2" in Amplitude und/oder Wellenlänge erfolgen kann. Ebenfalls ist es selbstverständlich denkbar, daß eine zusätzliche Modulationseinheit vorgesehen werden kann, die hier allerdings nicht weiter dargestellt ist, die die Meßstrahlung 8 selbst nach dem Verlassen der Strahlungsquellen 2, 2' und 2" moduliert. Zur Stabilisierung der Messungen wird gemäß der Figur 11 ein weiterer Strahlengang, der aus den Strahlteilern 48 austretenden Teilstrahlen über Spiegel 47 und einen Strahlteiler 48' aufgebaut, der in einen Referenzdetektor 49 einfällt und dort zur Kompensation von unerwünschten Effekten genutzt werden kann. Auch der Referenzdetektor 49 ist über Steuersignale 11' mit der Rechnereinheit 10 verbunden.

Die Figuren 4 bis 11 zeigen in einer sehr vereinfachten Darstellung einen gerätetechnischen Grundaufbau einer Vorrichtung nach den Ansprüche 12 bzw. 23. Hierbei beschränkt sich die Darstellung auf die wesentlichen Verfahrensabläufe und dazu notwendigen Gerätschaften. Es versteht sich von selbst, daß der Fachmann mit der erfindungsgemäßen Lehre entsprechend den Ansprüchen eine Vielzahl von Variationen und Anpassungen vornehmen kann, die ebenfalls vom Gegenstand der Erfindung umfaßt werden.

**Sachnummernliste**

| | |
|---|---|
| 1 - | Probengas |
| 2, 2', 2"- | Strahlungsquelle |
| 3 - | Probenzelle |
| 4 - | Auslaß |
| 5 - | Einlaß |
| 6 - | Modulationseinheit |
| 7 - | Strahlungsempfänger |
| 8 - | Meßstrahlung |
| 9 - | Meßsignale |
| 10 - | Rechnereinheit |
| 11 - | Steuersignale für Strahlungsquelle |
| 12 - | Steuersignale für Modulationseinheit |
| 13, 13' - | Steuersignale für Strahlungsempfänger |
| 14 - | Bewegungseinrichtung Strahlungsempfänger |
| 15 - | Strahlung vor Probenzelle |
| 16 - | Strahlung nach Probenzelle |

17 - Schwingbewegungsrichtung

18 - Gasleitung

19 - Lichtwellenleiter

20 - Ex-Barriere

21 - Interferenzfilter

22 - Abbildungsoptik

23 - Inertgas

24 - Kalibriergas

25 - Ventilblock

26 - Ansteuerung Probennahme

27 - Steuersignale für Chopper

28 - Chopper

29 - Drehrichtung Chopper

30 - Segmentöffnungen Chopper

31 - Spirale Chopper

32 - Monochromator

33 - Gehäuse

34 - erster optischer Wellenleiter

35 - zweiter optischer Wellenleiter

36 - erste Sektorelementgruppe

37 - zweite Sektorelementgruppe

38 - Y-Faserkoppler

39 - Hohlwellenleiter

40 - freie Wegstrecke

41 - Parallelstrahl

42 - sphärische Linse

43 - GRIND-Linse

44 - direkte Ankopplung

45 - Filterträger

46 - Blende

47 - Spiegel

48, 48' - Strahlteiler

49 - Referenzdetektor

**Patentansprüche**

1. Verfahren zur Bestimmung der Gasqualität eines Probengases (1), insbesondere eines Brenngases, ausgehend von einem unter Betriebsbedingungen mittels infrarotspektroskopischer Meßverfahren bestimmten Spektrums des Probengases (1),
   **dadurch gekennzeichnet, daß**

   - aus dem Spektrum die Stoffmengenanteile $x_i$ der Komponenten des Probengases (1) im Betriebszustand bestimmt werden,

   - Vorgabewerte für Kompressibilitätszahl K und Realgasfaktor $Z_n$ zur Berechnung der gesuchten Kompressibilitätszahl K vorgegeben werden,

   - aus Betriebsgrößen des Probengases (1) sowie den Stoffmengenanteilen $x_i$ und stoffspezifischen Größen und unter Einbeziehung der gewählten Vorgabewerte für Kompressibilitätszahl K und Realgasfaktor $Z_n$ Eingangsgrößen für die Bestimmung der Kompressibilitätszahl K bestimmt werden,

   - mit diesen Eingangsgrößen mittels Standard-Berechnungsverfahren die Kompressibilitätszahl K berechnet wird,

   - eine iterative Berechnung durch iterative Neuberechnung der Eingangsgrößen mit dem ermittelten Wert für die Kompressibilitätszahl K solange durchgeführt wird, bis der Wert der Kompressibilitätszahl K konvergiert und dann daraus der volumetrische Normbrennwert $H_{v,n}$ und die Normdichte $\rho_n$ berechnet wird.

**2.** Verfahren nach Anspruch 1, **dadurch gekennzeichnet, daß** als Standard-Berechnungsverfahren das Iterationsverfahren AGA8-92DC genutzt wird.

**3.** Verfahren nach Anspruch 1, **dadurch gekennzeichnet, daß** als Standard-Berechnungsverfahren das Iterationsverfahren GERG88 genutzt wird.

**4.** Verfahren nach einem der vorstehenden Ansprüche, **dadurch gekennzeichnet, daß** die Stoffmengenanteile $x_i$ der infrarotaktiven Komponenten des Probengases (1) im Betriebszustand aus dem aufgenommenen Spektrum mittels Multivariater Analyse (MVA) ermittelt werden.

**5.** Verfahren nach einem der vorstehenden Ansprüche, **dadurch gekennzeichnet, daß** die Vorgabewerte für Kompressibilitätszahl K und Realgasfaktor $Z_n$ aus einem Kennfeld entnommen werden, das den Einfluß von Betriebsdruck $p_b$ und Betriebstemperatur $T_b$ für eine bekannte, dem Probengas (1) ähnliche Gaszusammensetzung wiedergibt.

**6.** Verfahren nach einem der vorstehenden Ansprüche, **dadurch gekennzeichnet, daß** direkt aus dem Spektrum die Stoffmengen der infrarotaktiven Komponenten des Probengases im Betriebszustand und die Stoffmenge des Stickstoffes $N_2$ an dem Probengas (1) als Funktion der Stoffmengen der infrarotaktiven Komponenten des Probengases (1) ermittelt wird.

**7.** Verfahren nach Anspruch 6, **dadurch gekennzeichnet, daß** die Stoffmenge des Stickstoffes $N_2$ sich mit den Stoffmengen der infrarotaktiven Komponenten zum Gesamtvolumen des Probengases (1) ergänzt.

**8.** Verfahren zur Bestimmung der Gasqualität eines Probengases (1), insbesondere eines Brenngases, ausgehend von einem unter Betriebsbedingungen mittels infrarotspektroskopischer Meßverfahren bestimmten Spektrums des Probengases (1),
**dadurch gekennzeichnet, daß**

- Vorgabewerte für Kompressibilitätszahl K und Realgasfaktor $Z_n$ zur Berechnung der gesuchten Kompressibilitätszahl K vorgegeben werden,

- aus Betriebsdruck $p_b$ und Betriebstemperatur $T_b$ des Probengases (1) mit den aus dem Spektrum direkt bestimmbaren Werten für Betriebsbrennwert $H_{v,b}$ und Betriebsdichte $\rho_b$ Eingangsgrößen für die Bestimmung der Kompressibilitätszahl K berechnet werden,

- als weitere Eingangsgröße die molare Stoffmenge von $CO_2$ aus einer weiteren Absorptionsbande des Spektrums bestimmt wird,

- mit diesen Eingangsgrößen nach dem Iterationsverfahren GERG88 die Kompressibilitätszahl K berechnet wird,

- eine iterative Berechnung durch iterative Neuberechnung der Eingangsgrößen mit dem ermittelten Wert der Kompressibilitätszahl K solange durchgeführt wird, bis der Wert der Kompressibilitätszahl K konvergiert, und dann daraus der volumetrische Normbrennwert $H_{v,n}$ und die Normdichte $\rho_n$ berechnet wird.

**9.** Verfahren nach Anspruch 8, **dadurch gekennzeichnet, daß** Betriebsbrennwert $H_{v,b}$ und Betriebsdichte $\rho_b$ mittels spektraler Gewichtungsfunktionen direkt aus dem Spektrum des Probengases (1) bestimmt werden.

**10.** Verfahren nach Anspruch 9, **dadurch gekennzeichnet, daß** mit den spektralen Gewichtungsfunktionen der bewertete Einfluß der Stoffmengen der Komponenten des Probengases (1) auf den Betriebsbrennwert $H_{v,b}$ und die Betriebsdichte $\rho_b$ wiedergegeben wird.

**11.** Verfahren nach einem der Ansprüche 8 bis 10, **dadurch gekennzeichnet, daß** die Vorgabewerte für Kompressibilitätszahl K und Realgasfaktor $Z_n$ aus einem Kennfeld entnommen werden, das den Einfluß von Betriebsdruck $p_b$ und Betriebstemperatur $T_b$ für eine bekannte, dem Probengas (1) ähnliche Gaszusammensetzung wiedergibt.

**12.** Photometrische Vorrichtung zur Bestimmung des Transmissionsspektrums eines Probengases (1), insbesondere zur Durchführung eines der Verfahren nach Anspruch 1 oder Anspruch 8, aufweisend eine Meßstrahlung (8) erzeugende Strahlungsquelle (2), wobei die Meßstrahlung (8) eine Probenzelle (3) zur Aufnahme des Probengases (1) durchtritt und nach Durchtreten einer Modulationseinheit (6) zum Modulieren der Meßstrahlung (8) in mindestens einen Strahlungsempfänger (7) eintritt, der elektrische Meßsignale (9) entsprechend der einfallenden Intensität der Meßstrahlung (8) erzeugt und an eine elektronische Einheit (10) weiterleitet, die aus den Meßsignalen (9) ein Transmissionsspektrum ermittelt, wobei die Modulationseinheit (6) eine spektrale Schalteinheit (46, 47) in Form einer Chopperanordnung (28) aufweist, die aufgrund ihres selektiven Durchlaßverhaltens nur bestimmte Spektralbereiche des durch das Probengas (1) erzeugten Spektrums in der Meßstrahlung (8) zu dem Strahlungsempfänger (7) durchläßt.

**dadurch gekennzeichnet, daß**

die Chopperanordnung (28) eine Blende (46) mit einer spiralförmigen Öffnung (31) aufweist, bei der die Freigabe der Wellenlängenbereiche der Meßstrahlung (8) kontinuierlich über das ganze Spektrum erfolgt.

**13.** Photometrische Vorrichtung gemäß Anspruch 12, **dadurch gekennzeichnet, daß** die Chopperanordnung (28) ein derartiges Durchlaßverhalten aufweist, daß die durchgelassenen Spektralbereiche zur weiteren Auswertung von Verfahren der direkten Spektralauswertung (DSA) geeignet sind.

**14.** Photometrische Vorrichtung gemäß einem der Ansprüche 12 oder 13, **dadurch gekennzeichnet, daß** die durch die Chopperanordnung (28) jeweils durchgelassene Wellenlänge der Meßstrahlung (8) durch Erfassung der Drehstellung der Blende (46) ermittelbar ist.

**15.** Photometrische Vorrichtung gemäß einem der Ansprüche 12 bis 14, **dadurch gekennzeichnet, daß** die Chopperanordnung (28) zwei alternierend die Meßstrahlung (8) freigebende Sektorelementgruppen (36, 37) aufweist, wobei ein erster optischer Wellenleiter (34) die durch die Sektorelemente der ersten Sektorelementgruppe (36) freigegebene Meßstrahlung (8) in die Probenzelle (3) und nach dem Durchgang durch die Probenzelle (3) zu dem Strahlungsempfänger (7) und ein zweiter optischer Wellenleiter (35) die durch die Sektorelemente der zweiten Sektorelementgruppe (37) freigegebene Meßstrahlung (8) direkt zu dem Strahlungsempfänger (7) leitet.

**16.** Photometrische Vorrichtung gemäß Anspruch 15, **dadurch gekennzeichnet, daß** die durch die Sektorelemente der Sektorelementgruppen (36, 37) jeweils freigegebene Meßstrahlung (8) über ersten optischen Wellenleiter (34) und zweiten optischen Wellenleiter (35) in einem oder mehreren Filtern (21) oder einem dispersiven Element (6), vorzugsweise einem Monochromator (32), zusammengeführt sind.

**17.** Photometrische Vorrichtung gemäß Anspruch 16, **dadurch gekennzeichnet, daß** der Strahlungsempfänger (7) die aus dem einen oder den mehreren Filtern oder dem dispersiven Element austretende, durch die Sektorelemente der Sektorelementgruppen (36, 37) jeweils freigegebene Meßstrahlung (8) beider optischer Wellenleiter (34, 35) erfaßt.

**18.** Photometrische Vorrichtung gemäß einem der Ansprüche 15 bis 17, **dadurch gekennzeichnet, daß** die durch die Sektorelemente der Sektorelementgruppen (36, 37) jeweils freigegebene Meßstrahlung (8) desjenigen optischen Wellenleiters (34), der direkt zu dem Strahlungsempfänger (7) geleltet wird, als Referenz zum Eliminieren des Einflusses von in der Umgebung der Probenzelle (3) und/oder der Vorrichtung vorhandenem $CO_2$, Veränderungen der Strahlungsquelle (2) und/oder des Strahlungsempfängers (7) nutzbar ist.

**19.** Photometrische Vorrichtung gemäß einem der Ansprüche 15 bis 18, **dadurch gekennzeichnet, daß** die durch die Sektorelemente (30, 31) jeweils freigegebene Meßstrahlung (8) über ersten und zweiten optischen Wellenleiter (34, 35) dem Eingang des einen oder der mehreren Filter (21) oder des dispersiven Elementes (6) zugeführt sind, wobei an der Chopperanordnung (28) ebenfalls vorhandene weitere Sektorelementgruppen (36, 37) die aus dem einen oder den mehreren Filtern (21) oder dem dispersiven Element (6) austretende Meßstrahlung (8) alternierend auf den Strahlungsempfänger (7) aufschalten.

**20.** Photometrische Vorrichtung gemäß Anspruch 16 oder 19, **dadurch gekennzeichnet, daß** die durch die Sektorelemente (30, 31) jeweils freigegebene Meßstrahlung (8) über ersten und zweiten optischen Wellenleiter (34, 35) in einem Y-Faserkoppler (38) zusammengeführt sind, der die Meßstrahlung (8) des ersten und des zweiten optischen Wellenleiters (34, 35) dem einen oder den mehreren Filtern (21) oder dem dispersiven Element zuführt.

**21.** Photometrische Vorrichtung gemäß einem der Ansprüche 19 oder 20, **dadurch gekennzeichnet, daß** die Chopperanordnung (28) sowohl die Auswahl der Wellenlängen für das Spektrum als auch das alternierende Umschalten der Meßstrecke zwischen den Wellenleitern (34, 35) vornimmt.

**22.** Photometrische Vorrichtung gemäß einem der Ansprüche 15 bis 21, **dadurch gekennzeichnet, daß** die Probenzelle (3) mit einem infrarotinaktiven Gas, vorzugsweise Stickstoff $N_2$, spülbar ist, um eine Nullmessung zum Ausgleich von Verschmutzungen oder dgl. der optischen Einrichtungen (2, 7, 32) der Vorrichtung durchzuführen.

**Claims**

**1.** Procedure for the determination of the quality of gas of a probe gas (1), in particular a burnable gas, proceeding from a transmission spectrum of the probe gas (1) determined at operating conditions by means of spectroscopical methods of measurement,
**characterized in that**

- out of the spectrum the amounts of substances $x_i$ of the components of the probe gas (1) are determined at operating conditions,

- default values for compressibility factor K and real gas factor $Z_n$ are preset for calculation of the wanted compressibility factor K,

- out of quantities at operating conditions of the probe gas (1) as well as from the amounts of substances $x_i$ and substance specific quantities and taking into account of the selected default values for compressibility factor K and real gas factor $Z_n$ input quantities for the determination of the compressibility factor K are determined,

- with these input quantities the compressibility factor K is calculated by means of standard-arithmetic procedures,

- an iterative calculation in the way of an iterative recalculation of the input quantities is carried out with the determined value for the compressibility factor K as long, until the value of the compressibility factor K converges and than there from the volumetric standard calorific value $H_{v,n}$ and the standard density $\rho_n$ is calculated.

**2.** Procedure according to claim 1, **characterized in that** as standard-arithmetic procedure the method of iteration AGA8-92DC is used.

**3.** Procedure according to claim 1, **characterized in that** as standard-arithmetic procedure the method of iteration GERG88 is used.

**4.** Procedure according to one of the preceding claims, **characterized in that** the amounts of substances $x_i$ of the infrared active components of the probe gas (1) at operating conditions is determined starting from the recorded spectrum by means of multivariate analysis (MVA).

**5.** Procedure according to one of the preceding claims, **characterized in that** the default values of the compressibility factor K und the real gas factor $Z_n$ are taken from a characteristic diagram, that describes the influence of the pressure $p_b$ at operating conditions and the temperature $T_b$ at operating conditions for a known composition of a gas similar to the composition of the probe gas (1).

**6.** Procedure according to one of the preceding claims, **characterized in that** directly from the spectrum the amounts of substances of the infrared active components of the probe gas (1) at operating conditions and the amount of nitrogen $N_2$ of the probe gas (1) are determined as a function of the amounts of substances of the infrared active components of the probe gas (1).

**7.** Procedure according to claim 6, **characterized in that** the amount of substance of nitrogen $N_2$ and the amounts of substances of the infrared active components complements each other resulting in the total volume of the probe gas (1).

**8.** Procedure for the determination of the quality of gas of a probe gas (1), in particular a burnable gas, proceeding from a transmission spectrum of the probe gas (1) determined at operating conditions by means of spectroscopical methods of measurement,
**characterized in that**

- default values for compressibility factor K and real gas factor $Z_n$ are preset for calculation of the wanted compressibility factor K,

- from the pressure $p_b$ at operating conditions and the temperature $T_b$ at operating conditions of the probe gas (1) with the values for the calorific value $H_{v,b}$ at operating conditions and the density $\rho_b$ at operating conditions, which can be directly determined out of the spectrum, input quantities for the determination of the compressibility factor K are determined,

- as further input quantity the molar amount of substance of $CO_2$ is determined by means of a further absorption band of the spectrum,

- with these input quantities the compressibility factor K is calculated by means of the iterational procedure GERG88,

- an iterative calculation in the way of an iterative recalculation of the input quantities is carried out with the determined value for the compressibility factor K as long, until the value of the compressibility factor K converges and than there from the volumetric standard calorific value $H_{v,n}$ and the standard density $\rho_n$ is calculated.

9. Procedure according to claim 8, **characterized in that** the calorific value $H_{v,b}$ at operating conditions and the density $\rho_b$ at operating conditions are determined by means of spectral functions for weighting of a value directly from the spectrum of the probe gas (1).

10. Procedure according to claim 9, **characterized in that** with the spectral functions for weighting of a value the weighted influence of the amounts of substances of the components of the probe gas (1) is described for the calorific value $H_{v,b}$ at operating conditions and the density $\rho_b$ at operating conditions.

11. Procedure according to one of the claims 8 to 10, **characterized in that** the default values for compressibility factor K and real gas factor $Z_n$ are taken from a characteristic diagram, that describes the influence of the pressure $\rho_b$ at operating conditions and the temperature $T_b$ at operating conditions for a known composition of a gas similar to the composition of the probe gas (1).

12. Photometric device for the determination of a transmission spectrum of a probe gas (1), especially for carrying out one of the procedures according to claim 1 or claim 8, showing a radiation source (2) emitting a measurement radiation (8), in which the measurement radiation (8) passes through a probe cell (3) for capturing a probe gas (1) and enters after passing through a modulation unit (6) for modulating the measurement radiation (8) into at least one radiation receiver (7), which generates electrical measurement signals (9) according to the incoming intensity of the measurement radiation (8) and transmits these to an electronical unit (10), which determines a transmission spectrum out of the measurement signals (9), wherein the modulation unit (6) shows a spectral switch unit (46, 47) in the form of a chopper arrangement (28), which transmits because of their selective transmission behaviour only specific spectral regions of the spectrum in the measurement radiation (8) caused by the probe gas (1) to the radiation receiver (7).
**characterized in that**
the chopper arrangement (28) is provided with an aperture (46) with a spiral opening (31), in which the release of the regions of the wavelength of the measurement radiation (8) is caused continuously for the whole spectrum.

13. Photometric device according to claim 12, **characterized in that** the chopper arrangement (28) provides such a transmission behaviour, that the transmitted spectral regions are suitable for the further evaluation by procedures of the direct spectral evaluation (DSA).

14. Photometric device according to one of the claims 12 or 13, **characterized in that** the released wavelength of the measurement radiation (8), which passes through the chopper arrangement (28), can be obtained by means of capturing the rotational position of the aperture (46).

15. Photometric device according to one of the claims 12 to 14, **characterized in that** the chopper arrangement (28) is provided with two groups of sector elements (36, 37) alternatively releasing the measurement radiation (8), in which a first optical waveguide (34) guides the measurement radiation (8) released by the sector elements of the first sector element group (36) into the probe cell (3) and after passing through the probe cell (3) to the radiation receiver (7) and a second optical waveguide (35) guides the measurement radiation (8) released by the sector elements of the second sector element group (37) directly to the radiation receiver (7).

16. Photometric device according to claim 15, **characterized in that** the measurement radiation (8) released by the sector elements of the sector element groups (36, 37) are concentrated by means of first optical waveguide (34) and second optical waveguide (35) into one or more filters (21) or a dispersive element (6), preferably a monochromator (32).

17. Photometric device according to claim 16, **characterized in that** the radiation receiver (7) collects the measurement radiation (8), which is coming out of the one or more filters or the dispersive element and each released through the sector elements of the sector element groups (36, 37) of both optical waveguides (34, 35).

18. Photometric device according to one of the claims 15 to 17, **characterized in that** the measurement radiation (8), which is released through the sector elements of the sector element groups (36, 37) of that optical waveguide (34), which is guided directly to the radiation receiver (7), is usable as reference for eliminating the influence of $CO_2$, which exists in the surrounding of the probe cell (3) and/or of the device, of changes of the radiation source (2) and/or of the radiation receiver (7).

19. Photometric device according to one of the claims 15 to 18, **characterized in that** the measurement radiation (8), which is each released through the sector elements (30, 31), is guided through the first and the second waveguide (34, 35) to the input of the one or more filters (21) or the dispersive element (6), in which at the chopper arrangement (28) also available further sector element groups (36, 37) lock on the measurement radiation (8), which is released of the one or more filters (21) or the dispersive element (6), alternatively to the radiation receiver (7).

20. Photometric device according to claim 16 or 19, **characterized in that** the measurement radiation (8), which is each released through the sector elements (30, 31), is guided together by means of the first and the second waveguide (34, 35) in a Y-fibre coupler (38), which guides the measurement radiation (8) of the first and the second waveguide (34, 35) to the one or more filters (21) or the dispersive element (6).

21. Photometric device according to one of the claims 19 or 20, **characterized in that** the chopper arrangement (28) carries out both the selection of the wavelengths for the spectrum as well as the alternating reverse of the measured section between the waveguides (34, 35).

22. Photometric device according to one of the claims 15 to 21, **characterized in that** the probe cell (3) is sweepable with an infrared inactive gas, preferably nitrogen $N_2$, for carrying out a null measurement for the compensation of dirt accumulation or the same of the optical facilities (2, 7, 32) of the device.

**Revendications**

1. Procédé de détermination de la qualité d'un gaz de probe (1), en particulier d'un gaz combustible, partant d'un spectre du gaz de probe (1), determiné à l'aide des méthodes de mesurages spectroscopiques à infrarouge aux conditions de service,
   **caractérisé en ce que**

   - du spectre les rapports de quantités de substance en mol $x_i$ des componentes du gaz de probe (1) sont determinés aux conditions de service,

   - des valeurs directives pour le facteur de compressibilité K et le coefficient d'approximation pour les gaz réel $Z_n$ sont donnés pour la calculation du facteur de compressibilité K cherché,

   - des conditions de service du gaz de probe (1) ainsi que des rapports de quantités de substance en mol $x_i$ et des grandeurs specifiques pour les substances et inclusivement des valeurs directives cherchés pour le facteur de compressibilité K et le coefficient d'approximation pour les gaz réel $Z_n$ grandeurs d'entrées sont détermines pour la calculation du facteur de compressibilité K,

   - avec ces grandeurs d'entrées le facteur de compressibilité K est calculé avec des procédés de calculation standards,

- une calculation par iteration est executé avec recalculation par iteration des grandeurs d'entrées avec le valeur determiné pour le facteur de compressibilité K aussi longtemps, que le facteur de compressibilité K converge et puis de cela le pouvoir calorifique normal volumetrique $H_{v,n}$ et la densité à l'état normal $\rho_n$ est calculé.

**2.** Procédé selon la revendication 1, **caractérisé en ce que** comme procédé de calculation standard la méthode d'iteration AGA8-92DC est utilisée.

**3.** Procédé selon la revendication 1, **caractérisé en ce que** comme procédé de calculation standard la méthode d'iteration GERG88 est utilisée.

**4.** Procédé selon l'une quelconque des revendications précédentes, **caractérisé en ce que** des rapports de quantités de substance en mol $x_i$ des componentes actives en infrarouge du gaz de probe (1) aux conditions de service sont déterminés du spectre recepté avec la méthode d'analyse multivariable (MVA).

**5.** Procédé selon l'une quelconque des revendications précédentes, **caractérisé en ce que** des valeurs directives pour le facteur de compressibilité K et le coefficient d'approximation pour les gaz réel $Z_n$ sont pris d'un diagramme charactéristique, qui reproduit l'influence du pression de service $p_b$ et du température de service $T_b$ pour une composition du gaz connu, ressemblant au gaz de probe (1).

**6.** Procédé selon l'une quelconque des revendications précédentes, **caractérisé en ce que** du spectre les quantités de substance des componentes actives en infrarouge du gaz de probe (1) aux conditions de service et la quantité de substance du nitrogène $N_2$ du gaz de probe (1) en relation des quantités de substance des componentes actives en infrarouge du gaz de probe (1) sont déterminés directement.

**7.** Procédé selon la revendication 6, **caractérisé en ce que** la quantité de substance du nitrogène $N_2$ et les quantités de substance des componentes actives en infrarouge se compléte avec le volume total du gaz de probe (1).

**8.** Procédé de détermination de la qualité d'un gaz de probe (1), en particulier d'un gaz combustible, partant d'un spectre du gaz de probe (1), qui est determiné à l'aide des méthodes de mesurages spectroscopiques à infrarouge aux conditions de service,
**caractérisé en ce que**

- des valeurs directives pour le facteur de compressibilité K et le coefficient d'approximation pour les gaz réel $Z_n$ sont donnés pour la calculation du facteur de compressibilité K cherché,

- du pression de service $p_b$ et du température de service $T_b$ du gaz de probe (1) avec les valeurs pour le pouvoir calorifique aux conditions de service $H_{v,b}$ et la densité aux conditions de service $\rho_b$ grandeurs d'entrées sont détermines pour la calculation du facteur de compressibilité K,

- comme grandeurs d'entrées en plus la quantité de substance molaire du $CO_2$ est detérminée a la base du plusieurs bandes d'absorption du spectre,

- avec ces grandeurs d'entrées le facteur de compressibilité K est calculé par la méthode d'iteration GERG88,

- une calculation par iteration est executée avec recalculation par iteration des grandeurs d'entrées avec le valeur determiné pour le facteur de compressibilité K aussi longtemps, que le valeur de compressibilité K converge et puis de cela le pouvoir calorifique normale volumetrique $H_{v,n}$ et la densité à l'état normal $\rho_n$ est calculé.

**9.** Procédé selon la revendication 8, **caractérisé en ce que** le pouvoir calorifique aux conditions de service $H_{v,b}$ et la densité aux conditions de service $\rho_b$ sont calculés à l'aide du functions de pondération spectrales directement du spectre du gaz de probe (1).

**10.** Procédé selon la revendication 9, **caractérisé en ce que** avec les functions de pondération spectrales l'influence évalué des quantités de substance des componentes du gaz de probe (1) au pouvoir calorifique aux conditions de service $H_{v,b}$ et la densité aux conditions de service $\rho_b$ est répresentée.

**11.** Procédé selon l'une quelconque des revendications 8 à 10, **caractérisé en ce que** des valeurs directives pour le facteur de compressibilité K et le coefficient d'approximation pour les gaz réel $Z_n$ sont pris d'un diagramme caractéristique, qui reproduit l'influence du pression de service $p_b$ et du température de service $T_b$ pour une composition du gaz connu, ressemblant au gaz de probe (1).

**12.** Dispositif photometrique pour la detérmination d'un spectre de transmission d'un gaz de probe (1), en particulier pour la realisation d'une des procédés selon la revendication 1 ou la revendication 8, presentant une source de rayonnement (2), qui produit une radiation de mesure (8), où la radiation de mesure (8) passe une cellule d'échantillon (3) pour la réception du gaz de probe (1) et après le passage d'un dispositif à la modulation (6) pour la modulation de la radiation de mesure (8) entre dans au moins un radiorécepteur (7), qui produit signales de mesure électriques (9) correspondant à l'intensité pénétrante de la radiation de mesure (8) et transmet ces signales à un dispositif électronique (10), qui détermine un spectre de transmission de ces signales de mesure électriques (9), où le dispositif à modulation (6) présent un dispositif à connecter (46, 47) en forme d'un arrangement des vibreurs (28), qui en raison de son attitude de transmission sélective ne laisse passer que domains spectrales specifique du spectre dans la radiation de mesure (8), produit au gaz de probe (1), sur le radiorécepteur (7),
**caractérisé en ce que**
l'arrangement des vibreurs (28) présent un cadre (46) avec un orifice spiral (31), par lequel le relâchement des domaines de longueur d'onde du radiation de mesure (8) résulte continuellement du tous le spectre.

**13.** Dispositif photometrique selon la revendication 12, **caractérisé en ce que** l'arrangement des vibreurs (28) présent une attitude de transmission de telle manière, que des domaines de longueur d'onde laissent passé sont approprié pour l'interprétation suivante par procédés de l'évaluation spectrale directe (DSA).

**14.** Dispositif photometrique selon l'une quelconque des revendications 12 ou 13, **caractérisé en ce que** la longueur actuellement d'onde de la radiation de mesure (8), laissent passé par l'arrangement des vibreurs (28), est déterminable par détermination du position pivotant du cadre (46).

**15.** Dispositif photometrique selon l'une quelconque des revendications 12 à 14, **caractérisé en ce que** l'arrangement des vibreurs (28) présent deux groupes des elements sectorals (36, 37), qui affranchisent alternant la radiation de mesure (8), òu un premier guide d'onde optique (34) conduit la radiation de mesure (8), qui est affranchi par des elements sectorals du premier groupe des elements sectorals (36), sur la cellule d'échantillon (3) et après le passage par la cellule d'échantillon (3) au radiorécepteur (7), et un deuxième guide d'onde optique (35) conduit la radiation de mesure (8), qui est affranchi par des elements sectorals du deuxième groupe des elements sectorals (37) directement au radiorécepteur (7).

**16.** Dispositif photometrique selon la revendication 15, **caractérisé en ce que** la radiation de mesure (8), qui est affranchi actuellement par des elements sectorals des groupes des elements sectorals (36, 37), sont concentrés par le premier guide d'onde optique (34) et le deuxième guide d'onde optique (35) dans un ou plusieurs filtres (21) ou un élément dispersif, en particulier un monochromateur (32).

**17.** Dispositif photometrique selon la revendication 6, **caractérisé en ce que** le radiorécepteur (7) saisit la radiation de mesure (8), qui est sortie d'un ou du plusieurs filtres (21) ou d'un élément dispersif et qui est affranchi actuellement par des elements sectorals des groupes des elements sectorals (36, 37) des deux guides d'onde optiques (34, 35).

**18.** Dispositif photometrique selon l'une quelconque des revendications 15 à 17, **caractérisé en ce que** la radiation de mesure (8), qui est affranchi actuellement par des elements sectorals des groupes des elements sectorals (36, 37) et conduit directement au radiorécepteur (7) par ce guide d'onde optique (34), est utilisable comme référence pour éliminer l'influence de $CO_2$, qui est existant en environs de la cellule d'échantillon (3) et/ou du dispostif, des changements de la source de rayonnement (2) et/ou du radiorécepteur (7).

**19.** Dispositif photometrique selon l'une quelconque des revendications 15 à 18, **caractérisé en ce que** la radiation de mesure (8), qui est affranchi actuellement par des elements sectorals (30, 31) par le premier et le deuxième guide d'onde optique (34, 35) est conduit à l'entrée d'un ou du plusieurs filtres (21) ou du élément dispersif, óu à l'arrangement des vibreurs (28) aussi autres groupes existantes des elements sectorals (36, 37) donnent la radiation de mesure (8), qui émerge d'un ou du plusieurs filtres (21) ou du élément dispersif, alternant au radiorécepteur (7).

**20.** Dispositif photometrique selon revendications 16 ou 19, **caractérisé en ce que** la radiation de mesure (8), qui est affranchi actuellement par des elements sectorals (30, 31) par le premier et le deuxième guide d'onde optique (34, 35), est concentrée dans un Y-coupleur à fibre (38), qui conduit la radiation de mesure (8) du premier et du deuxième guide d'onde optique (34, 35) à un ou plusieurs filtres (21) ou à l'élément dispersif.

21. Dispositif photometrique selon l'une quelconque des revendications 19 ou 20, **caractérisé en ce que** l'arrangement des vibreurs (28) fait non seulement le changement des longueurs d'onde pour le spectre mais aussi la commutation alternante de section mesurée entre des guides d'onde optiques (34, 35).

22. Dispositif photometrique selon l'une quelconque des revendications 15 à 21, **caractérisé en ce que** la cellule d'échantillon (3) est lavable avec un gaz inactive en infrarouge, en particulier nitrogène $N_2$, pour exécuter un mesure zéro pour la compensation des pollutions ou de même des installations optiques (2, 7, 32) du dispositif.

Spektren maßgeblicher Gaskomponenten bei 8bar

EP 1 141 677 B1

## Fig. 2a

```
┌─────────────────────────────────┐
│      Multivariate Analyse       │
└─────────────────────────────────┘
                 │
                 ▼
┌─────────────────────────────────┐
│       Ansatz für K und Zn       │
└─────────────────────────────────┘
                 │
                 ▼
┌─────────────────────────────────┐
│ Berechnung der Eingangs-        │
│ größen für die Realgas-         │
│ berechnung                      │
│ p, T, xi für AGA8-92DC          │
│ p, T, Hₙ, ρn, XCO₂ für GERG88   │
└─────────────────────────────────┘
          │                 │
          ▼                 ▼
┌──────────────┐    ┌──────────────┐
│  AGA8-92DC   │    │   GERG88     │
└──────────────┘    └──────────────┘
```

Berechnung der Eingangsgrößen für die Realgasberechnung
$p$, $T$, $x_i$ für AGA8-92DC
$p$, $T$, $H_n$, $\rho_n$, $X_{CO_2}$ für GERG88

Konvergenz für K ?  nein  mit ermitteltem K iterieren

Berechnung von $x_i$, $H_{v,n}$, $\rho_n$
aus AGA8-92DC oder
Berechnung von $H_{v,n}$, $\rho_n$, $X_{CO_2}$
aus GERG88

## Fig. 2b

Direkte Spektralauswertung

Ansatz für K und $Z_n$

Berechnung der Eingangsgrößen für die Realgasberechnung

$p$, $T$, $H_n$, $\rho_n$, $X_{CO_2}$ für GERG88

GERG88

Konvergenz für K ?  nein  mit ermitteltem K iterieren

Berechnung von
$H_{v,n}$, $\rho_n$, $X_{CO_2}$
aus GERG88

Fig. 3a

Fig. 3b

EP 1 141 677 B1

Stand der Technik

EP 1 141 677 B1

Fig. 4

Fig. 5

$I_0(\lambda)$

$I(\lambda)$

EP 1 141 677 B1

Fig. 6

$I_0(\lambda)$

$I(\lambda)$

EP 1 141 677 B1

Fig. 7

Fig. 8

34

4    8    39

34

EP 1 141 677 B1

5

Fig. 9

Fig. 10a

Stand der Technik

Fig. 10b

$I_0(\lambda 1)$    2      2'

$I_0(\lambda 2)$

21'    21"

21

$I_0(\lambda 3)$   2"

GAS

1

15

48    48

7

8

47    49

48'

13'    11

13

10

EP 1 141 677 B1